(19) Europäisches Patentamt
European Patent Office
Office européen des brevets

(11) EP 1 934 198 B1

(12) **EUROPEAN PATENT SPECIFICATION**

(45) Date of publication and mention of the grant of the patent:
**03.08.2011 Bulletin 2011/31**

(21) Application number: **06803516.1**

(22) Date of filing: **13.09.2006**

(51) Int Cl.:
***C07D 401/04*** *(2006.01)* ***A61P 3/04*** *(2006.01)*
***A61K 31/513*** *(2006.01)*

(86) International application number:
**PCT/US2006/035701**

(87) International publication number:
**WO 2007/035372 (29.03.2007 Gazette 2007/13)**

(54) **POLYMORPHS OF BENZOATE SALT OF 2-[[6-[(3R)-3-AMINO-1-PIPERIDINYL]-3,4-DIHYDRO-3-METHYL-2,4-DIOXO-1(2H)-PYRIMIDINYL]METHYL]-BENZONITRILE AND METHODS OF USE THEREFOR**

POLYMORPHE DES BENZOATSALZES VON 2-[[6-[(3R)-3-AMINO-1-PIPERIDINYL]-3,4-DIHYDRO-3-METHYL-2,4-DIOXO-1(2H)-PYRIMIDINYL]METHYL]-BENZONITRIL UND VERWENDUNGSVERFAHREN DAFÜR

FORMES POLYMORPHES DE SEL BENZOATE DE 2-[[6-[(3R)-3-AMINO-1-PIPERIDINYL]-3,4-DIHYDRO-3-METHYL-2,4-DIOXO-1(2H)-PYRIMIDINYL]METHYL]-BENZONITRILE, ET LEURS PROCEDES D'UTILISATION

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **16.09.2005 US 718133 P**

(43) Date of publication of application:
**25.06.2008 Bulletin 2008/26**

(73) Proprietor: **Takeda Pharmaceutical Company Limited**
**Osaka-shi,**
**Osaka 541-0045 (JP)**

(72) Inventors:
- **ANDRES, Mark**
  **West Lafayette, IN 47906 (US)**
- **LORIMER, Keith**
  **West Lafayette, IN 47906 (US)**

(74) Representative: **Duncan, Garreth Andrew et al**
**D Young & Co LLP**
**120 Holborn**
**London**
**EC1N 2DY (GB)**

(56) References cited:
**EP-A- 1 586 571** **EP-A- 1 586 571**
**WO-A1-2005/095381** **US-A1- 2005 070 535**

- **CAIRA M R: "CRYSTALLINE POLYMORPHISM OF ORGANIC COMPOUNDS" TOPICS IN CURRENT CHEMISTRY, SPRINGER, BERLIN, DE, vol. 198, 1998, pages 163-208, XP001156954 ISSN: 0340-1022**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 934 198 B1

Printed by Jouve, 75001 PARIS (FR)

## Description

### FIELD OF THE INVENTION

The present invention relates generally to polymorphs of the benzoate salt of 2-[[6-[(3R)-3-amino-1-piperidinyl]-3,4-dihydro-3-methyl-2,4-dioxo-1 (2H)-pyrimidinyl]methyl]-benzonitrile (referred to herein as "Compound I"); compositions, kits and articles of manufacture comprising polymorphs of Compound I; and their use.

### DESCRIPTION OF RELATED ART

Dipeptidyl Peptidase IV (IUBMB Enzyme Nomenclature EC.3.4.14.5) is a type II membrane protein that has been referred to in the literature by a wide a variety of names including DPP4, DP4, DAP-IV, FAPβ adenosine deaminase complexing protein 2, adenosine deaminase binding protein (ADAbp), dipeptidyl aminopeptidase IV; Xaa-Pro-dipeptidyl-aminopeptidase; Gly-Pro naphthylamidase; postproline dipeptidyl aminopeptidase IV; lymphocyte antigen CD26; glycoprotein GP110; dipeptidyl peptidase IV; glycylproline aminopeptidase; glycylproline aminopeptidase; X-prolyl dipeptidyl aminopeptidase; pep X; leukocyte antigen CD26; glycylprolyl dipeptidylaminopeptidase; dipeptidyl-peptide hydrolase; glycylprolyl aminopeptidase; dipeptidyl-aminopeptidase IV; DPP IV/CD26; amino acyl-prolyl dipeptidyl aminopeptidase; T cell triggering molecule Tp103; X-PDAP. Dipeptidyl Peptidase IV is referred to herein as "DPP-IV".

DPP-IV is a non-classical serine aminodipeptidase that removes Xaa-Pro dipeptides from the amino terminus (N-terminus) of polypeptides and proteins. DPP-IV dependent slow release of dipeptides of the type X-Gly or X-Ser has also been reported for some naturally occurring peptides.

DPP-IV is constitutively expressed on epithelial and endothelial cells of a variety of different tissues (intestine, liver, lung, kidney and placenta), and is also found in body fluids. DPP-IV is also expressed on circulating T-lymphocytes and has been shown to be synonymous with the cell-surface antigen, CD-26. DPP-IV has been implicated in a number of disease states, some of which are discussed below.

DPP-IV is responsible for the metabolic cleavage of certain endogenous peptides (GLP-1 (7-36), glucagon) *in vivo* and has demonstrated proteolytic activity against a variety of other peptides (GHRH, NPY, GLP-2, VIP) *in vitro.*

GLP-1 (7-36) is a 29 amino-acid peptide derived by post-translational processing of proglucagon in the small intestine. GLP-1 (7-36) has multiple actions *in vivo* including the stimulation of insulin secretion, inhibition of glucagon secretion, the promotion of satiety, and the slowing of gastric emptying. Based on its physiological profile, the actions of GLP-1 (7-36) are believed to be beneficial in the prevention and treatment of type II diabetes and potentially obesity. For example, exogenous administration of GLP-1 (7-36) (continuous infusion) in diabetic patients has been found to be efficacious in this patient population. Unfortunately, GLP-1 (7-36) is degraded rapidly *in vivo* and has been shown to have a short half-life *in vivo* ($t_{1/2}$=1.5 minutes).

Based on a study of genetically bred DPP-IV knock out mice and on *in vivo* / *in vitro* studies with selective DPP-IV inhibitors, DPP-IV has been shown to be the primary degrading enzyme of GLP-1 (7-36) *in vivo.* GLP-1 (7-36) is degraded by DPP-IV efficiently to GLP-1 (9-36), which has been speculated to act as a physiological antagonist to GLP-1 (7-36). Inhibiting DPP-IV *in vivo* is therefore believed to be useful for potentiating endogenous levels of GLP-1 (7-36) and attenuating the formation of its antagonist GLP-1 (9-36). Thus, DPP-IV inhibitors are believed to be useful agents for the prevention, delay of progression, and/or treatment of conditions mediated by DPP-IV, in particular diabetes and more particularly, type 2 diabetes mellitus, diabetic dislipidemia, conditions of impaired glucose tolerance (IGT), conditions of impaired fasting plasma glucose (IFG), metabolic acidosis, ketosis, appetite regulation and obesity.

DPP-IV expression is increased in T-cells upon mitogenic or antigenic stimulation (Mattem, T., et al., Scand. J. Immunol., 1991, 33, 737). It has been reported that inhibitors of DPP-IV and antibodies to DPP-IV suppress the proliferation of mitogen-stimulated and antigen-stimulated T-cells in a dose-dependant manner (Schon, E., et al., Biol. Chem., 1991, 372, 305). Various other functions of T-lymphocytes such as cytokine production, IL-2 mediated cell proliferation and B-cell helper activity have been shown to be dependent on DPP-IV activity (Schon, E., et al., Scand. J. Immunol., 1989, 29, 127). DPP-IV inhibitors, based on boroProline, (Flentke, G. R., et al., Proc. Nat. Acad. Sci. USA, 1991, 88, 1556) although unstable, were effective at inhibiting antigen-induced lymphocyte proliferation and IL-2 production in murine CD4+ T-helper cells. Such boronic acid inhibitors have been shown to have an effect *in vivo* in mice causing suppression of antibody production induced by immune challenge (Kubota, T. et al., Clin. Exp. Immun., 1992, 89, 192). The role of DPP-IV in regulating T lymphocyte activation may also be attributed, in part, to its cell-surface association with the transmembrane phosphatase, CD45. DPP-IV inhibitors or non-active site ligands may possibly disrupt the CD45-DPP-IV association. CD45 is known to be an integral component of the T-cell signaling apparatus. It has been reported that DPP-IV is essential for the penetration and infectivity of HIV-1 and HIV-2 viruses in CD4+ T-cells (Wakselman, M., Nguyen, C., Mazaleyrat, J.-P., Callebaut, C., Krust, B., Hovanessian, A. G., Inhibition of HIV-1 infection of CD 26+ but not CD 26-cells by a potent cyclopeptidic inhibitor of the DPP-IV activity of CD 26. Abstract P.44 of the 24th European Peptide Symposium 1996). Additionally, DPP-IV has been shown to associate with the enzyme adenosine

deaminase (ADA) on the surface of T-cells (Kameoka, J., et al., Science, 193, 26 466). ADA deficiency causes severe combined immunodeficiency disease (SCID) in humans. This ADA-CD26 interaction may provide clues to the pathophysiology of SCID. It follows that inhibitors of DPP-IV may be useful immunosuppressants (or cytokine release suppressant drugs) for the treatment of among other things: organ transplant rejection; autoimmune diseases such as inflammatory bowel disease, multiple sclerosis and rheumatoid arthritis; and the treatment of AIDS.

It has been shown that lung endothelial cell DPP-IV is an adhesion molecule for lung-metastatic rat breast and prostate carcinoma cells (Johnson, R. C., et al., J. Cell. Biol., 1993, 121, 1423). DPP-IV is known to bind to fibronectin and some metastatic tumor cells are known to carry large amounts of fibronectin on their surface. Potent DPP-IV inhibitors may be useful as drugs to prevent metastases of, for example, breast and prostrate tumors to the lungs.

High levels of DPP-IV expression have also been found in human skin fibroblast cells from patients with psoriasis, rheumatoid arthritis (RA) and lichen planus (Raynaud, F., et al., J. Cell. Physiol., 1992, 151, 378). Therefore, DPP-IV inhibitors may be useful as agents to treat dermatological diseases such as psoriasis and lichen planus.

High DPP-IV activity has been found in tissue homogenates from patients with benign prostate hypertrophy and in prostatosomes. These are prostate derived organelles important for the enhancement of sperm forward motility (Vanhoof, G., et al., Eur. J. Clin. Chem. Clin. Biochem., 1992, 30, 333). DPP-IV inhibitors may also act to suppress sperm motility and therefore act as a male contraceptive agent. Conversely, DPP-IV inhibitors have been implicated as novel for treatment of infertility, and particularly human female infertility due to Polycystic ovary syndrome (PCOS, Stein-Leventhal syndrome) which is a condition characterized by thickening of the ovarian capsule and formation of multiple follicular cysts. It results in infertility and amenorrhea.

DPP-IV is thought to play a role in the cleavage of various cytokines (stimulating hematopoietic cells), growth factors and neuropeptides.

Stimulated hematopoietic cells are useful for the treatment of disorders that are characterized by a reduced number of hematopoietic cells or their precursors *in vivo.* Such conditions occur frequently in patients who are immunosuppressed, for example, as a consequence of chemotherapy and/or radiation therapy for cancer. It was discovered that inhibitors of dipeptidyl peptidase type IV are useful for stimulating the growth and differentiation of hematopoietic cells in the absence of exogenously added cytokines or other growth factors or stromal cells. This discovery contradicts the dogma in the field of hematopoietic cell stimulation, which provides that the addition of cytokines or cells that produce cytokines (stromal cells) is an essential element for maintaining and stimulating the growth and differentiation of hematopoietic cells in culture. (See, e.g., PCT Intl. Application No. PCT /US93/017173 published as WO 94/03055).

DPP-IV in human plasma has been shown to cleave N-terminal Tyr-Ala from growth hormone-releasing factor and cause inactivation of this hormone. Therefore, inhibitors of DPP-IV may be useful in the treatment of short stature due to growth hormone deficiency (Dwarfism) and for promoting GH-dependent tissue growth or regrowth.

DPP-IV can also cleave neuropeptides and has been shown to modulate the activity of neuroactive peptides substance P, neuropeptide Y and CLIP (Mentlein, R., Dahms, P., Grandt, D., Kruger, R., Proteolytic processing of neuropeptide Y and peptide YY by dipeptidyl peptidase IV, Regul. Pept., 49, 133, 1993; Wetzel, W., Wagner, T., Vogel, D., Demuth, H.-U., Balschun, D., Effects of the CLIP fragment ACTH 20-24 on the duration of REM sleep episodes, Neuropeptides, 31, 41, 1997). Thus DPP-IV inhibitors may also be useful agents for the regulation or normalization of neurological disorders.

A need still exists for DPP-IV inhibitors that have advantageous potency, stability, selectivity, toxicity and/or pharmacodynamics properties and which thus may be used effectively in pharmaceutical compositions to treat disease states by the inhibition of DPP-IV.

**SUMMARY OF THE INVENTION**

The benzoate salt of 2-[[6-[(3R)-3-amino-1-piperidinyl]-3,4-dihydro-3-methyl-2,4-dioxo-1(2H)-pyrimidinyl]methyl]-benzonitrile (referred to herein as Compound I) which has the formula:

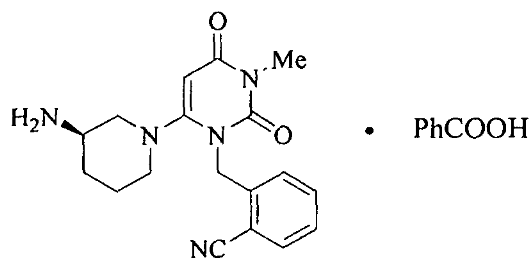

is a DPP-IV inhibitor that is described in U.S. Patent Application Serial No. 11/080,992, filed March 15, 2005.

The present invention provides compositions comprising one or more polymorphs of Compound I. For ease of reference, the polymorphs described herein are referred to consistently as Form A and amorphous Form 1.

**1. FORM A**

In one embodiment, the present invention relates to a method for preparing a polymorph of Compound I, referred to herein as Form A. Based on its physical properties, Form A is a crystalline form.

Form A may be characterized as having one or more of the following physical characteristics (it being noted that a composition need not necessarily exhibit all of these characteristics in order to indicate the presence of Form A):

(a) may be formed by crystallization from any of the following solvent systems (i) acetone, (ii) acetonitrile; (iii) butanol, (iv) dimethylsulfoxide; (v) dioxane; (vi) ethanol; (vii) ethanol and isopropyl alcohol; (viii) ethanol and water; (ix) ethyl acetate; (x) heptane; (xi) isopropanol; (xii) isopropyl acetate; (xiii) methanol; (xiv) methyl ethyl ketone; (xv) methyl isobutyl ketone; (xvi) 2,2,2-trifluoroethanol; (xvii) tetrahydrofuran; (xviii) toluene; (xix) water; and (xx) ethanol and heptane.

(b) has an X-ray powder diffraction pattern with salient features being major diffraction lines as shown below:

| | | | | | | | | | | | |
|---|---|---|---|---|---|---|---|---|---|---|---|
| **°2θ** | 9.12 | 9.44 | 10.48 | 10.84 | 11.34 | 12.49 | 12.84 | 14.09 | 14.38 | 14.90 | 15.20 |
| **$I/I_o$** | 7 | 56 | 3 | 28 | 10 | 8 | 3 | 7 | 5 | 10 | 27 |
| | | | | | | | | | | | |
| **°2θ** | 16.83 | 17.48 | 17.82 | 18.75 | 20.09 | 20.48 | 20.64 | 20.92 | 21.18 | 21.52 | 21.82 |
| **$I/I_o$** | 3 | 4 | 50 | 100 | 21 | 5 | 20 | 83 | 51 | 17 | 34 |
| | | | | | | | | | | | |
| **°2θ** | 22.10 | 22.88 | 23.34 | 23.64 | 23.88 | 24.22 | 24.44 | 25.87 | 26.14 | 27.02 | 27.62 |
| **$I/I_o$** | 14 | 9 | 11 | 20 | 9 | 7 | 12 | 16 | 4 | 28 | 16 |
| | | | | | | | | | | | |
| **°2θ** | 28.09 | 28.52 | 29.06 | 29.26 | 29.74 | 30.17 | 31.66 | 33.02 | 34.34 | 34.86 | 35.12 |
| **$I/I_o$** | 13 | 25 | 14 | 9 | 8 | 3 | 4 | 3 | 12 | 7 | 11 |
| | | | | | | | | | | | |
| **°2θ $I/I_o$** | 35.50 | 36.07 | 37.32 | 37.52 | 37.82 | 38.02 | 38.29 | | | | |
| | 8 | 8 | 3 | 4 | 9 | 9 | 15 | | | | |

and, in particular, having the following distinguishing peaks:

| **Peak Position (°2θ)** | **$I/I_o$** |
|---|---|
| 9.44 | 56 |
| 10.84 | 28 |
| 17.82 | 50 |
| 18.75 | 100 |
| 25.87 | 16 |
| 28.52 | 25 |

(c) has an IR spectrum comprising absorption peaks at 830, 876, 910, 950, 987, 1004, 1026, 1063, 1094, 1135, 1173, 1212, 1231, 1284, 1316, 1334, 1365, 1384, 1447, 1458, 1474, 1532, 1592, 1613, 1697, 2082, 2230, 2540, 2596, 2743, 2860, 2958, 2979 and 3085 $cm^{-1}$;

with an IR spectrum comprising unique FT-IR peak positions (peaks that show no other peak within ± 4 $cm^{-1}$ to make up a unique set) at 1212, 1365, 1447, 1613 and 1697 $cm^{-1}$;

(d) has FT-Raman peak positions at 825, 881, 910, 918, 987, 1003, 1027, 1039, 1065, 1084, 1103, 1135, 1157, 1167, 1172, 1184, 1206, 1235, 1288, 1337, 1365, 1385, 1417, 1446, 1461, 1474, 1557, 1577, 1597, 1624 1652, 1689, 2230, 2860, 2883, 2957, 2970, 2983, 3026, 3053 and 3070 $cm^{-1}$;

with unique FT-Raman peak positions (peaks that show no other peaks within ± 4 $cm^{-1}$ to make up a unique set) at 1065, 1103, 1235, 1288, 1337, 1365, 1624, 1689, 2883, 2983 and 3026 $cm^{-1}$;
(e) has a differential scanning calorimetry spectrum having an endotherm range of about 173 °C to about 195 °C, optionally an endotherm range of about 180 °C to about 190 °C, and optionally an endotherm at 186 °C;
(f) has a thermogravimetric analysis data showing a 0.2% weight loss from 26 -159 °C; and/or
(g) formed from the conversion of the amorphous Form 1 by stressing the amorphous Form 1 with heat, high relative humidity or organic vapors, or by wet milling of amorphous Form 1 with water.

## 2. AMORPHOUS FORM 1

The amorphous Form 1 may be characterized as having one or more of the following physical characteristics (it being noted that a composition need not necessarily exhibit all of these characteristics in order to indicate the presence of the amorphous Form 1):

(a) may be formed by (i) rotoevaporation from methanol; (ii) fast evaporation from water; (iii) lyophilization from water; (iv) crystallization from ethyl acetate and hexanes; and (v) crystallization from isopropyl acetate and hexanes;
(b) has an X-ray powder diffraction pattern that shows a broad halo with no specific peaks present;
(c) has an IR spectrum comprising absorption peaks at 809, 833, 868, 948, 1024, 1068, 1084, 1119, 1134, 1172, 1228, 1286, 1375, 1440, 1541, 1599, 1652, 1703, 2136, 2225, 2571, 2861, 2949 and 3062 $cm^{-1}$;
with an IR spectrum comprising unique FT-IR peak positions (peaks that show no other peaks within ± 4 $cm^{-1}$ to make up a unique set) at 809, 868, 1119, 1599 and 1703 $cm^{-1}$;
(d) has FT-Raman peak position at 805, 834, 904, 1002, 1024, 1045, 1134, 1168, 1205, 1280, 1386, 1443, 1578, 1600, 1654, 1703, 2225, 2864, 2958 and 3065 $cm^{-1}$;
with unique FT-Raman peak positions (peaks that show no other peaks within ± 4 $cm^{-1}$) at 805, 1280 and 1703 $cm^{-1}$;
(e) has a differential scanning calorimetry (cyclic DSC) spectrum having a Tg = 70 °C (onset), exotherm at 132 °C (maxima), and an endotherm at 183 °C (onset temperature); and/or
(f) has a thermogravimetric analysis data showing a 4% weight loss from 25-151 °C.

Methods by which the above referenced analyses were performed in order to identify these physical characteristics are described in the Examples.
The present invention relates to compositions comprising Compound I, wherein Compound I is present as the amorphous Form 1, as described below. It is noted that other crystalline and amorphous forms of Compound I may also be present in the composition.
In one variation, the composition comprises at least 0.25%, 0.5%, 1%, 5%, 10%, 25%, 50%, 75%, 80%, 85%, 90%, 95%, 97%, or 99% of Compound I where greater than 1%, 5%, 10%, 25%, 50%, 75%, 80%, 85%, 90%, 95%, 97% or 99% of Compound I (by weight) is present in the composition as the amorphous Form 1. The composition may optionally be a pharmaceutical composition. The pharmaceutical composition may optionally further include one or more pharmaceutical carriers.
Also provided are kits and other articles of manufacture comprising a composition that comprises Compound I, wherein Compound I is present as the amorphous Form 1. In one variation, the composition comprises at least 0.25%, 0.5%, 1%, 5%, 10%, 25%, 50%, 75%, 80%, 85%, 90%, 95%, 97%, or 99% of Compound I where greater than 1%, 5%, 10%, 25%, 50%, 75%, 80%, 85%, 90%, 95%, 97% or 99% of Compound I (by weight) is present in the composition as the amorphous Form 1. The composition in the kits and articles of manufacture may optionally be a pharmaceutical composition. The pharmaceutical composition may optionally further include one or more pharmaceutical carriers.
In regard to each of the above embodiments including a pharmaceutical composition, the pharmaceutical composition may be formulated in any manner where a portion of the compound is at least partially preserved in a given polymorphic form. Optionally, a portion of the compound is at least partially preserved in a given polymorphic form for a period of time subsequent to administration of the pharmaceutical formulation to a human.

## 3. Methods of Making Form A and Amorphous Forms 1

Various methods are also provided for making Form A and amorphous Form 1. Various methods are also provided for manufacturing pharmaceutical compositions, kits and other articles of manufacture comprising Form A and amorphous Form 1.

## 4. Use of Amorphous Form 1

Use of a pharmaceutical composition, kit and other article of manufacture comprising amorphous Form 1 in

the manufacture of a medicament to treat various diseases are also provided.

In another embodiment, the invention provides a composition comprising amorphous Form 1 of Compound I (as defined above) for use in treating diabetes and more particularly, type 2 diabetes mellitus, diabetic dislipidemia, conditions of impaired glucose tolerance (IGT), conditions of impaired fasting plasma glucose (IFG), metabolic acidosis, ketosis, appetite regulation and obesity.

## BRIEF DESCRIPTION OF THE FIGURES

Figure 1 illustrates the XRPD pattern of Form A, wherein the "XRPD pattern" is a plot of the intensity of diffracted lines.

Figure 2 is a plot of TGA data and the DSC data for Form A.

Figure 3 is a plot of the IR absorption spectrum for Form A.

Figure 4 is a plot of the FT-Raman absorption spectrum for Form A.

Figure 5 illustrates the XRPD pattern of amorphous Form 1, wherein the "XRPD pattern" is a plot of the intensity of diffracted lines.

Figure 6 is a plot of TGA data and the DSC data for amorphous Form 1.

Figure 7 is a plot of the IR absorption spectrum for amorphous Form 1.

Figure 8 is a plot of the FT-Raman absorption spectrum for amorphous Form 1.

## DETAILED DESCRIPTION OF THE INVENTION

The present invention provides compositions comprising Compound I where at least a portion of Compound I is present in the composition as amorphous Form 1.

Also provided are kits and other articles of manufacture with compositions comprising Compound I where at least a portion of Compound I is present in the composition as amorphous Form 1.

Various methods are also provided including methods of making the disclosed Form A and amorphous Form 1, methods for manufacturing pharmaceutical compositions comprising Compound I where at least a portion of Compound I is present in the composition as amorphous Form 1, and use of compositions comprising Compound I where at least a portion of Compound I is present in the composition as amorphous Form 1.

### 1. Preparation of Compound I

Various methods may be used to synthesize Compound I. Representative methods for synthesizing Compound I are provided in Example 1. It is noted, however, that other synthetic routes may also be used to synthesize Compound I including those disclosed in U.S. Patent Application Serial No. 11/080,992, filed March 15, 2005.

### 2. Preparation of Polymorphs

In general, a given polymorph of a compound may be obtained by direct crystallization of the compound or by crystallization of the compound followed by interconversion from another polymorphic form or from an amorphous state. The Examples below describe methods for testing the solubility of Compound I and methods for screening for crystallization conditions for Compound I.

Depending on the method by which a compound is crystallized, the resulting composition may contain different amounts of the compound in crystalline form as opposed to as an amorphous material. Also, the resulting composition may contain differing mixtures of different polymorphic forms of the compound.

"Crystalline", as the term is used herein, refers to a material that contains a specific compound, which may be hydrated and/or solvated, and has sufficient crystalline content to exhibit a discernable diffraction pattern by XRPD or other diffraction techniques. Often, a crystalline material that is obtained by direct crystallization of a compound dissolved in a solution or interconversion of crystals obtained under different crystallization conditions, will have crystals that contain the solvent used in the crystallization, termed a crystalline solvate. Also, the specific solvent system and physical embodiment in which the crystallization is performed, collectively termed crystallization conditions, may result in the crystalline material having physical and chemical properties that are unique to the crystallization conditions, generally due to the orientation of the chemical moieties of the compound with respect to each other within the crystal and/or the predominance of a specific polymorphic form of the compound in the crystalline material.

Depending upon the polymorphic form(s) of the compound that are present in a composition, various amounts of the compound in an amorphous solid state may also be present, either as a side product of the initial crystallization, and/or a product of degradation of the crystals comprising the crystalline material. Thus, crystalline, as the term is used herein, contemplates that the composition may include amorphous content; the presence of the crystalline material

among the amorphous material being detectably among other methods by the composition having a discernable diffraction pattern.

The amorphous content of a crystalline material may be increased by grinding or pulverizing the material, which is evidenced by broadening of diffraction and other spectral lines relative to the crystalline material prior to grinding. Sufficient grinding and/or pulverizing may broaden the lines relative to the crystalline material prior to grinding to the extent that the XRPD or other crystal specific spectrum may become undiscernable, making the material substantially amorphous or quasi-amorphous.

"Amorphous", as the term is used herein, refers to a composition comprising a compound that contains too little crystalline content of the compound to yield a discernable pattern by XRPD or other diffraction techniques. Glassy materials are a type of amorphous material. Amorphous materials do not have a true crystal lattice, and are consequently glassy rather than true solids, technically resembling very viscous non-crystalline liquids. Rather than being true solids, glasses may better be described as quasi-solid amorphous material. Thus, an amorphous material refers to a quasi-solid, glassy material. Precipitation of a compound from solution, often affected by rapid evaporation of solvent, is known to favor the compound forming an amorphous solid as opposed to crystals. A compound in an amorphous state may be produced by rapidly evaporating solvent from a solvated compound, or by grinding, pulverizing or otherwise physically pressurizing or abrading the compound while in a crystalline state. General methods for precipitating and crystallizing a compound may be applied to prepare the various polymorphs described herein. These general methods are known to those skilled in the art of synthetic organic chemistry and pharmaceutical formulation, and are described, for example, by J. March, "Advanced Organic Chemistry: Reactions, Mechanisms and Structure," 4th Ed. (New York: Wiley-Interscience, 1992).

"Broad" or "broadened", as the term is used herein to describe spectral lines, including XRPD, NMR and IR spectroscopy lines, is a relative term that relates to the line width of a baseline spectrum. The baseline spectrum is often that of an unmanipulated crystalline form of a specific compound as obtained directly from a given set of physical and chemical conditions, including solvent composition and properties such as temperature and pressure. For example, broadened can be used to describe the spectral lines of a XRPD spectrum of ground or pulverized material comprising a crystalline compound relative to the material prior to grinding. In materials where the constituent molecules, ions or atoms, as solvated or hydrated, are not tumbling rapidly, line broadening is indicative of increased randomness in the orientation of the chemical moieties of the compound, thus indicative of an increased amorphous content. When comparisons are made between crystalline materials obtained via different crystallization conditions, broader spectral lines indicate that the material producing the relatively broader spectral lines has a higher level of amorphous material.

Continued grinding would be expected to increase the amorphous content and further broaden the XRPD pattern with the limit of the XRPD pattern being so broadened that it can no longer be discerned above noise. When the XRPD pattern is broadened to the limit of being indiscernible, the material may be considered to no longer be a crystalline material, and instead be wholly amorphous. For material having increased amorphous content and wholly amorphous material, no peaks should be observed that would indicate grinding produces another form.

Compositions comprising a higher percentage of crystalline content (*e.g.*, forming crystals having fewer lattice defects and proportionately less glassy material) are generally prepared when conditions are used that favor slower crystal formation, including those slowing solvent evaporation and those affecting kinetics. Crystallization conditions may be appropriately adjusted to obtain higher quality crystalline material as necessary. Thus, for example, if poor crystals are formed under an initial set of crystallization conditions, the solvent temperature may be reduced and ambient pressure above the solution may be increased relative to the initial set of crystallization conditions in order to slow crystallization.

As one will appreciate, depending on how a composition comprising a given compound is produced and then, once produced, how the composition is stored and manipulated, will influence the crystalline content of the composition. Accordingly, it is possible for a composition to comprise no crystalline content or may comprise higher concentrations of crystalline content.

It is further noted that a compound may be present in a given composition in one or more different polymorphic forms, as well as optionally also being present as an amorphous material. This may be the result of (a) physically mixing two or more different polymorphic forms; (b) having two or more different polymorphic forms be generated from crystallization conditions; (c) having all or a portion of a given polymorphic form convert into another polymorphic form; (d) having all or a portion of a compound in an amorphous state convert into two or more polymorphic forms; as well as for a host of other reasons.

As can be seen, depending on how a composition comprising a compound is prepared, the percentage, by weight, of that compound in a given polymorphic form can vary from 0% to 100%. According to the present invention, compositions are provided where greater than 1%, 5%, 10%, 25%, 50%, 75%, 80%, 85%, 90%, 95%, 97%, or 99% or more of Compound I (by weight) is present in the composition as amorphous Form 1.

### 3. Polymorphs of Compound I

Described herein are Form A and amorphous Form 1 of Compound I.

Various tests may be performed in order to physically characterize the crystalline state of Compound I including but not limited to X-ray powder diffraction ("XRPD"), differential scanning calorimetry ("DSC"), thermogravimetric analysis ("TGA"), hot stage microscopy, infrared spectrometry ("IR"), Raman spectrometry and Karl Fischer analysis. The Examples below describe the methods that were used to perform the various analyses reported herein. Where possible, the results of each test for each different polymorph are provided herein.

### A. Method of Making Form A of Compound I

The following describes procedures by which Form A polymorph of Compound I has been made from different samples of Compound I:

Sample No. 1924-73-02: A slurry of Compound I in acetone was filtered through 0.2 μm nylon syringe filter into a clean vial. The vial was left uncovered in a fume hood under ambient conditions for fast evaporation, yielding Form A solids after two days.

Sample No. 1924-73-08: A slurry of Compound I in methanol was filtered through 0.2 μm nylon syringe filter into a clean vial. The vial was covered with aluminum foil perforated with pinholes and placed in a fume hood for slow evaporation at ambient conditions, yielding Form A solids in two days.

Sample No. 1924-67-05: Compound I was slurried in acetonitrile on a hotplate set at 60 °C and the mixture was filtered while warm through 0.2 μm nylon syringe filter into a clean, warm vial. The vial was placed on the hotplate, which was then turned off and allowed to slowly cool to ambient temperature. Form A solids were collected by filtration after one day.

Sample No. 1994-82-01: Compound I (123 mg) was dissolved in 99:1 ethanol/isopropyl acetate (1 mL) under reflux and then was cooled to ambient temperature at a rate of 20 °C/hour. Solids precipitated and the resulting slurry was agitated for 4 hours at ambient temperature. The solvent was decanted and the solids dried. The experiments yielded Form A solids.

### B. Characterization of Form A of Compound I

Figure 1 illustrates the XRPD pattern of Form A. Major diffraction lines are observed for °2θ at approximately: 9.44, 10.84, 17.82, 18.75, 25.87 and 28.52. The XRPD pattern tends to indicate that confirmed that the material is a crystalline phase, which was designated form A.

Colorless plates of Compound I were collected from ethanol-water solution. The orthorhombic cell parameters and calculated volume are: a = 8.0869(2), b = 9.9030(3), c = 28.5471(10) Å, V = 2286.18(12) $Å^3$. For Z = 4 and formula weight of 461.53g, the calculated density is 1.34 $g/cm^3$. The quality of the structure obtained is good, as indicated by the R-value of 0.068. Usually R-values in the range of 0.02 to 0.06 are quoted for the most reliably determined structures. An ORTEP drawing of Compound I shows that the crystal structure is the same as the proposed structure. The asymmetric unit was noted to contain one free base cation and one benzoate counterion. The calculated powder pattern generated from the single crystal data with experimental XRPD patterns of Form A of Compound I are very similar, but there are small differences in °2θposition of peaks due to temperature effects. The single crystal data was collected at 150K whereas the experimental XRPD pattern was measured at ambient temperature. Differences in intensities are likely due to preferred orientation.

Thermogravimetric and DSC data for Form A is summarized below in Table 1A and plotted in Figure 2. As can be seen in Figure 2, the DSC curve exhibits several endothermic events. The endotherm maximum for the most predominant event is located near 186 °C. A melting point experiment confirmed that this endothermic event is associated with the melt of the material. The series of endothermic events above the melt endotherm were not characterized further, but likely correspond to the decomposition of the sample. A broad endothermic event located below the melt endotherm for the sample can also be seen in the DSC plot for the sample of form A. This event occurs in the same temperature region as the corresponding mass loss observed in the TGA plot for a sample of form A and is consistent with the loss of volatile material from the sample.

**TABLE 1A**

| Thermal Data for Form A | |
|---|---|
| DSC Results [1] | TGA Results [2] |
| Endotherm at 186 °C | 0.2% |

[1] Maximum temperature reported for transition
[2] Percent weight change from 26 °C to 159 °C

The IR spectrum for Form A (Lot no. QZ-656-17(1)) is plotted in Figure 3.

The Raman spectrum for Form A (Lot no. QZ-656-17(1)) is provided in Figure 4.

Data regarding the moisture sorption/desorption properties of Form A are summarized in Table 2A below. The analysis showed an initial weight gain of approximately 0.035% from ambient conditions to 55% RH and a weight loss of about 0.05% between 9% and 95% RH. The desorption cycle follows a slightly different path as the sorption cycle, where the mass gained during the sorption cycle is lost upon desorption in slightly amounts at the various RH intervals. The weight change value for the desorption cycle can be used along with the molecular weight of Compound I to calculate that no molecule of water is present in the crystal at 95% RH. This calculation assumes that no water is present in the sample at the 5% RH step at the end of the analysis. The post moisture sorption/desorption sample was identified as form A by XRPD. In separate experiments form A was stressed under various RH conditions at room temperature (Table 3A). After several weeks none of these samples had undergone a phase change. This is evident in the respective XRPD patterns for the samples, which all correspond to form A. The gradual mass increase/decrease observed throughout the range of RH levels examined in the moisture sorption/desorption profile coupled with the lack of a phase change for form A upon RH stress indicates that this solid phase may contain a variable amount of water that will be dependent on the RH of the environment. This type of hydrated phase is referred to as a variable or non-stoichiometric hydrate.

**TABLE 2A**

| Elapsed Time (min) | Weight (mg) | Weight chg (%) | Sample Temp (°C) | Sample RH (%) |
|---|---|---|---|---|
| 0.1 | 12.164 | 0.000 | 25.20 | 9.14 |
| 14.1 | 12.164 | 0.002 | 25.20 | 5.18 |
| 21.1 | 12.164 | 0.001 | 25.22 | 14.96 |
| 28.2 | 12.164 | 0.001 | 25.20 | 24.97 |
| 35.2 | 12.164 | 0.002 | 25.20 | 35.05 |
| 42.2 | 12.164 | 0.004 | 25.20 | 44.89 |
| 51.2 | 12.168 | 0.035 | 25.19 | 54.85 |
| 60.2 | 12.168 | 0.039 | 25.21 | 64.93 |
| 69.2 | 12.169 | 0.041 | 25.20 | 74.86 |
| 78.2 | 12.169 | 0.045 | 25.21 | 84.87 |
| 87.2 | 12.170 | 0.049 | 25.21 | 94.56 |
| 97.0 | 12.166 | 0.019 | 25.21 | 85.20 |
| 105.7 | 12.166 | 0.017 | 25.21 | 75.06 |
| 114.7 | 12.165 | 0.016 | 25.21 | 65.04 |
| 123.7 | 12.165 | 0.014 | 25.21 | 55.13 |
| 131.7 | 12.165 | 0.012 | 25.22 | 45.01 |
| 139.7 | 12.165 | 0.008 | 25.21 | 34.97 |
| 146.7 | 12.168 | 0.035 | 25.22 | 24.96 |
| 153.7 | 12.167 | 0.032 | 25.21 | 14.97 |
| 163.7 | 12.163 | -0.004 | 25.20 | 4.82 |

**TABLE 3A. Compound I Polymorph Screen - Solids-Based Experiments: Stress Studies using Glassy Materials**

| Starting sample | Sample No. | Stress[a] | Duration | Habit[b] | XRPD File | Result |
|---|---|---|---|---|---|---|
| 1924-83-01[c] | 1924-83-04 | 81% RH | 9 days | colorless glass containing small, fine needles | - | - |
| 1924-83-02[c] | 1924-83-05 | 40°C | 9 days | small blades | 100971 | form A |
| 1924-83-03[c] | 1924-83-06 | 79% RH/ 40°C | 9 days | fine needles | 100972 | form A |
| 1966-09-01[c] | 1966-27-01 | 79% RH/ 60°C | 1 hr | opaque irregularly shaped solids, unknown morphology | 109327 | form A |
| | 1966-27-02 | 79% RH/ 60°C | 3 hrs | opaque irregularly shaped solids, unknown morphology | 109328[c] | form A |
| | 1966-27-03 | 79% RH/ 60°C | 6.5 hrs | opaque irregularly shaped solids, unknown morphology | - | - |

a. RH = relative humidity.
b. Observations made visually or using polarized light microscopy.
c. Samples considered non-GMP.

**C. Method of Making Amorphous Form 1 of Compound I**

Sample No. 1994-26-01: A slurry of Compound I was placed on a hot plate set at 80 °C. The mixture was filtered through 0.2 μm nylon syringe filter into a warm vial. The vial was then placed on the hot plate, which was then set to 40 °C and the solution allowed to cool. Sufficient hexanes were added to cause a cloudy suspension to form. Fine solids were collected by filtration and allowed to air dry. The experiments yielded amorphous solids.

Sample No. 1994-07-01: Sufficient Compound I was added to methanol such that undissolved solids remained. The resulting slurry was filtered through 0.2 μm nylon syringe filter into a flask. The solution was evaporated to dryness using a rotary evaporator (Buchi, R-114) under reduced pressure. The amorphous solids were stored in desiccator.

**D. Characterization of Amorphous Form 1 of Compound I**

**Polymorph Amorphous Form 1:**

Figure 5 illustrates the XRPD pattern of amorphous Form 1 (Sample No. 1994-12-01). The XRPD data show poor signal-to-noise ratio. The XRPD pattern tends to indicate that Form 1 is an amorphous form of Compound I.

Thermogravimetric and DSC data for amorphous Form 1 are summarized below in Table 1B and plotted in Figure 6. Amorphous Form 1 was prepared by several methods as noted above. The material was very hygroscopic gaining 10% weight under 85% RH but lost weight above 85% RH, which is indicative of crystallization. The post moisture balance XRPD pattern matched form A. TGA weight loss was 4% between 25-168 °C, probably due to adsorbed moisture. Therefore a cyclic DSC experiment was performed to dry the sample and then determine the glass transition temperature $T_g$, the onset of which was 70 °C. An exothermic recrystallization event was recorded at 132 °C, followed by a sharp endotherm at 183 °C (onset), which correlated with the onset of the melt (172 °C) determined by hot stage microscopy. This suggests that the amorphous solids crystallized to form A during heating.

**TABLE 1B**

| Thermal Data for Amorphous Form 1 | |
|---|---|
| Cyclic DSC Results | TGA Results |
| $T_g$ = 70°C (onset) [1],<br>Exotherm maxima at 132 °C,<br>Endotherm onset at 183°C | 4 % from<br>25-151°C |
| [1] $T_g$ = glass transition temperature | |

The IR and FT-Raman spectra for amorphous Form 1 (sample no. 1994-07-01) are plotted in Figures 7 and 8, respectively.

Data regarding the moisture sorption/desorption properties of amorphous Form 1 are summarized in Table 2B below. The moisture sorption/desorption data show an initial weight loss of approximately 1 % from ambient conditions to 5% RH and then a weight gain of about 6.5% between 5% and 95% RH. Between 85% and 95% RH the sample lost about 5% of weight, which is suggestive of a crystallization event. The sample recovered after the moisture sorption/desorption experiment was tentatively identified as containing form A.

**TABLE 2B**

| Elapsed Time (min) | Weight (mg) | Weight chg (%) | Sample Temp (°C) | Sample RH (°C) |
|---|---|---|---|---|
| 0.1 | 10.660 | 0.000 | 24.98 | 53.89 |
| 151.0 | 10.559 | -0.953 | 25.00 | 5.25 |
| 197.3 | 10.612 | -0.456 | 25.00 | 14.81 |
| 253.1 | 10.663 | 0.025 | 24.99 | 24.80 |
| 329.5 | 10.721 | 0.569 | 24.98 | 34.93 |
| 508.3 | 10.838 | 1.669 | 24.99 | 44.84 |
| 603.1 | 11.053 | 3.680 | 24.98 | 54.90 |
| 655.1 | 11.277 | 5.782 | 24.97 | 64.85 |
| 723.7 | 11.559 | 8.432 | 24.98 | 74.54 |
| 764.6 | 11.740 | 10.125 | 24.98 | 84.54 |
| 952.3 | 11.244 | 5.474 | 24.95 | 94.62 |
| 1139.9 | 11.017 | 3.348 | 24.98 | 85.49 |
| 1327.5 | 10.864 | 1.908 | 24.98 | 75.25 |
| 1517.0 | 10.730 | 0.655 | 24.98 | 65.12 |
| 1706.6 | 10.615 | -0.421 | 25.00 | 55.16 |
| 1894.1 | 10.532 | -1.208 | 25.01 | 45.08 |
| 2081.7 | 10.475 | -1.737 | 24.99 | 34.87 |
| 2199.9 | 10.453 | -1.946 | 24.99 | 24.87 |
| 2221.4 | 10.449 | -1.980 | 24.99 | 15.16 |
| 2238.3 | 10.446 | -2.007 | 24.99 | 5.06 |

### 4. Pharmaceutical Compositions Comprising Compound I Where at Least Amorphous Form 1 is Present

The polymorphs of the present invention may be used in various pharmaceutical compositions. Such pharmaceutical compositions may comprise Compound I present in the composition in a range of between 0.005% and 100% (weight/weight), with the balance of the pharmaceutical composition comprising additional substances such as those described herein. In particular variations, the pharmaceutical composition comprises at least 0.25%, 0.5%, 1%, 5%, 10%, 25%, 50%, 75%, 80%, 85%, 90%, 95%, 97%, or 99% of Compound I. A given one of the polymorphic forms of Compound I may comprise at least 1%, 5%, 10%, 25%, 50%, 75%, 80%, 85%, 90%, 95%, 97%, or 99% of Compound I (weight/weight) in the pharmaceutical composition.

In general, the pharmaceutical compositions of the present invention may be prepared in a gaseous, liquid, semi-liquid, gel, or solid form, and formulated in a manner suitable for the route of administration to be used where at least a portion of Compound I is present in the composition as amorphous Form 1.

Pharmaceutical compositions according to the present invention may be adapted for administration by any of a variety of routes. For example, pharmaceutical compositions according to the present invention can be administered orally, parenterally, intraperitoneally, intravenously, intraarterially, transdermally, sublingually, intramuscularly, rectally, transbuccally, intranasally, liposomally, via inhalation, vaginally, intraoccularly, via local delivery (for example, by catheter or stent), subcutaneously, intraadiposally, intraarticularly, or intrathecally, optionally in a slow release dosage form. In particular embodiments, the pharmaceutical compounds are administered orally, by inhalation or by injection subcutaneously, intramuscularly, intravenously or directly into the cerebrospinal fluid.

In addition to Compound I, the pharmaceutical composition may comprise one or more additional components that do not deleteriously affect the use of Compound I. For example, the pharmaceutical compositions may include, in addition to Compound I, conventional pharmaceutical excipients; diluents; lubricants; binders; wetting agents; disintegrating agents; glidants; sweetening agents; flavoring agents; emulsifying agents; solubilizing agents; pH buffering agents; perfuming agents; surface stabilizing agents; suspending agents; and other conventional, pharmaceutically inactive agents. In particular, the pharmaceutical compositions may comprise lactose, sucrose, dicalcium phosphate, carboxymethylcellulose, magnesium stearate, calcium stearate, talc, starch, natural gums (*e.g.*, gum acaciagelatin, glucose, molasses, polyvinylpyrrolidine, celluloses and derivatives thereof), povidone, crospovidones acetate, sodium citrate, cyclodextrine derivatives, sorbitan monolaurate, triethanolamine sodium acetate, triethanolamine oleate, and other such agents. Methods of preparing such dosage forms are known in the art, and will be apparent to those skilled in this art; for example, see Remington's Pharmaceutical Sciences, 19th Ed. (Easton, Pa.: Mack Publishing Company, 1995). The pharmaceutical composition to be administered should, in any event, contain a sufficient quantity of Compound I to reduce dipeptidyl peptidases activity *in vivo* sufficiently to provide the desired therapeutic effect.

Compositions, according to the present invention, may be administered, or coadministered with other active agents. These additional active agents may include, for example, one or more other pharmaceutically active agents. Coadministration in the context of this invention is intended to mean the administration of more than one therapeutic agent, one of which includes Compound I. Such coadministration may also be coextensive, that is, occurring during overlapping periods of time or may be sequential, that is, occurring during non-overlapping periods of time.

The following are particular examples of oral, intravenous and tablet formulations that may optionally be used with Compound I. It is noted that these compositions may be varied depending on the indication for which the composition is to be used.

Exemplary capsule formulations are as follows:

**12.5mg of Compound I (weight of free base form) per tablet**

Core Tablet Formulation

| | | |
|---|---|---|
| (1) | 2-[[6-[(3R)-3-amino-1-piperidinyl]-3,4-dihydro-3-methyl-2,4-dioxo-1 (2H)-pyrimidinyl]methyl]-benzonitrile (benzoate salt) | 17.0mg |
| (2) | Lactose Monohydrate, NF, Ph, Eur (FOREMOST 316 FAST FLO) | 224.6 mg |
| (3) | Microcrystalline Cellulose, NF, Ph, Eur (AVICEL PH 102) | 120.1 mg |
| (4) | Croscarmellose Sodium, NF, Ph, Eur (AC-DI-SOL) | 32.0 mg |
| (5) | Colloidal Silicon Dioxide, NF, Ph, Eur (CAB-O-SIL M-5P) | 3.2 mg |
| (6) | Magnesium Stearate, NF, Ph, Eur (MALLINCKRODT, Non-bovine Hyqual) | 3.2 mg |
| TOTAL (per tablet) | | 400.0 mg |

Film Coat (12.0 mg in total)

(1) Opadry II 85F18422, White - Portion 1 (COLORCON)

(2) Opadry II 85F18422, White - Portion 2 (COLORCON)

(3) Opadry II 85F18422, White - Portion 3 (COLORCON)

**25mg of Compound I (weight of free base form) per tablet**

Core Tablet Formulation

| | | |
|---|---|---|
| (1) | 2-[[6-[(3R)-3-amino-1-piperidinyl]-3,4-dihydro-3-methyl-2,4- dioxo-1 (2H)-pyrimidinyl]methyl]-benzonitrile (benzoate salt) | 34.0 mg |
| (2) | Lactose Monohydrate, NF, Ph, Eur (FOREMOST 316 FAST FLO) | 207.6 mg |
| (3) | Microcrystalline Cellulose, NF, Ph, Eur (AVICEL PH 102) | 120.1 mg |
| (4) | Croscarmellose Sodium, NF, Ph; Eur (AC-DI-SOL) | 32.0 mg |
| (5) | Colloidal Silicon Dioxide, NF, Ph, Eur (CAB-O-SIL M-5P) | 3.2 mg |

(continued)

| Core Tablet Formulation | | |
|---|---|---|
| (6) | Magnesium Stearate, NF, Ph, Eur (MALLINCKRODT, Non-bovine Hyqual) | 3.2 mg |
| TOTAL (per tablet) | | 400.0 mg |
| Film Coat (12.0 mg in total) | | |
| | (1) Opadry II 85F18422, White - Portion 1 (COLORCON) | |
| | (2) Opadry II 85F18422, White - Portion 2 (COLORCON) | |
| | (3) Opadry II 85F18422, White - Portion 3 (COLORCON) | |

**50mg of Compound I (weight of free base form) per tablet**

| Core Tablet Formulation | | |
|---|---|---|
| (1) | 2-[[6-[(3R)-3-amino-1-piperidinyl]-3,4-dihydro-3-methyl-2,4-dioxo-1 (2H)-pyrimidinyl]methyl]-benzonitrile (benzoate salt) | 68.0 mg |
| (2) | Lactose Monohydrate, NF, Ph, Eur (FOREMOST 316 FAST FLO) | 173.6 mg |
| (3) | Microcrystalline Cellulose, NF, Ph, Eur (AVICEL PH 102) | 120.1 mg |
| (4) | Croscarmellose Sodium, NF, Ph, Eur (AC-DI-SOL) | 32.0 mg |
| (5) | Colloidal Silicon Dioxide, NF, Ph, Eur (CAB-O-SIL M-5P) | 3.2 mg |
| (6) | Magnesium Stearate, NF, Ph, Eur (MALLINCKRODT, Non-bovine Hyqual) | 3.2 mg |
| TOTAL (per tablet) | | 400.0 mg |
| Film Coat (12.0 mg in total) | | |
| | (1) Opadry II 85F18422, White - Portion 1 (COLORCON) | |
| | (2) Opadry II 85F18422, White - Portion 2 (COLORCON) | |
| | (3) Opadry II 85F18422, White - Portion 3 (COLORCON) | |

Exemplary intravenous and tablet formulations are as follows:

| INTRAVENOUS FORMULATION | |
|---|---|
| Compound of the Present Invention | 0.1-10 mg |
| Dextrose Monohydrate | q.s. to make isotonic |
| Citric Acid Monohydrate | 1.05 mg |
| Sodium Hydroxide | 0.18 mg |
| Water for Injection | q.s. to 1.0 mL |

| TABLET FORMULATION | |
|---|---|
| Compound of the Present Invention | 1% |
| Microcrystalline Cellulose | 73% |
| Stearic Acid | 25% |
| Colloidal Silica | 1% |

Provided in the examples are, by way of illustration but not limitation, more particular examples of formulations incorporating one or more of Form A or amorphous Form 1.

### 5. Indications for Use of Compound I

Use of Compound I and compositions, kits and articles of manufacture comprising Compound I to inhibit DPP-IV is described herein. Use of Compound I and compositions, kits and articles of manufacture comprising Compound I to treat a disease state for which DPP-IV possesses activity that contributes to the pathology and/or symptomology of the disease state is also described herein.

Compound I may be administered to a subject wherein DPP-IV activity within the subject is altered, preferably

reduced.

Examples of diseases that may be treated by use of Compound I and compositions according to the present invention include, but are not limited to diabetes, more particular type 2 diabetes mellitus, diabetic dislipidemia, conditions of impaired glucose tolerance (IGT), conditions of impaired fasting plasma glucose (IFG), metabolic acidosis, ketosis, appetite regulation, obesity, immunosuppressants or cytokine release regulation, autoimmune diseases such as inflammatory bowel disease, multiple sclerosis and rheumatoid arthritis, AIDS, cancers (prevention of metastases, for example, breast and prostrate tumors to the lungs), dermatological diseases such as psoriasis and lichen planus, treatment of female infertility, osteoporosis, male contraception and neurological disorders.

## 6. Kits and Articles of Manufacture Comprising Compound I Polymorphs

The present invention is also directed to kits and other articles of manufacture for use in treating diseases associated with dipeptidyl peptidases. It is noted that diseases are intended to cover all conditions for which the dipeptidyl peptidases possesses activity that contributes to the pathology and/or symptomology of the condition.

In one embodiment, a kit is provided that comprises a pharmaceutical composition comprising Compound I where greater than 1%, 5%, 10%, 25%, 50%, 75%, 80%, 85%, 90%, 95%, 97% or 99% of Compound I (by weight) is present in the composition as amorphous Form 1; and instructions for use of the kit. Optionally, the composition comprises at least 0.25%, 0.5%, 1%, 5%, 10%, 25%, 50%, 75%, 80%, 85%, 90%, 95%, 97%, or 99% of Compound I. The instructions may indicate the disease state for which the composition is to be administered, storage information, dosing information and/or instructions regarding how to administer the composition. The kit may also comprise packaging materials. The packaging material may comprise a container for housing the composition. The kit may also optionally comprise additional components, such as syringes for administration of the composition. The kit may comprise the composition in single or multiple dose forms.

In another embodiment, an article of manufacture is provided that comprises a pharmaceutical composition comprising Compound I where greater than 1%, 5%, 10%, 25%, 50%, 75%, 80%, 85%, 90%, 95%, 97% or 99% of Compound I (by weight) is present in the composition as amorphous Form 1; and packaging materials. Optionally, the composition comprises at least 0.25%, 0.5%, 1%, 5%, 10%, 25%, 50%, 75%, 80%, 85%, 90%, 95%, 97%, or 99% of Compound I. The packaging material may comprise a container for housing the composition. The container may optionally comprise a label indicating the disease state for which the composition is to be administered, storage information, dosing information and/or instructions regarding how to administer the composition. The article of manufacture may also optionally comprise additional components, such as syringes for administration of the composition. The article of manufacture may comprise the composition in single or multiple dose forms.

It is noted that the packaging material used in kits and articles of manufacture according to the present invention may form a plurality of divided containers such as a divided bottle or a divided foil packet. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. The container that is employed will depend on the exact dosage form involved, for example a conventional cardboard box would not generally be used to hold a liquid suspension. It is feasible that more than one container can be used together in a single package to market a single dosage form. For example, tablets may be contained in a bottle that is in turn contained within a box. Typically the kit includes directions for the administration of the separate components. The kit form is particularly advantageous when the separate components are preferably administered in different dosage forms (*e.g.*, oral, topical, transdermal and parenteral), are administered at different dosage intervals, or when titration of the individual components of the combination is desired by the prescribing physician.

One particular example of a kit according to the present invention is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

Another specific embodiment of a kit is a dispenser designed to dispense the daily doses one at a time in the order of their intended use. Preferably, the dispenser is equipped with a memory-aid, so as to further facilitate compliance

with the regimen. An example of such a memory-aid is a mechanical counter that indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

**7. Dosage Forms**

Compositions according to the present invention are optionally provided for administration to humans and animals in unit dosage forms or multiple dosage forms, such as tablets, capsules, pills, powders, dry powders for inhalers, granules, sterile parenteral solutions or suspensions, oral solutions or suspensions, and oil-water emulsions containing suitable quantities Compound I. Unit-dose forms, as used herein, refers to physically discrete units suitable for human and animal subjects and packaged individually as is known in the art. Each unit-dose contains a predetermined quantity of Compound I sufficient to produce the desired therapeutic effect, in association with a pharmaceutical carrier, vehicle or diluent. Examples of unit-dose forms include ampoules and syringes, and individually packaged tablets or capsules. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules, or bottles of pints or gallons. Hence, multiple dose form may be viewed as a multiple of unit-doses that are not segregated in packaging.

In general, the total amount of Compound I in a pharmaceutical composition according to the present invention should be sufficient to a desired therapeutic effect. This amount may be delivered as a single per day dosage, multiple dosages per day to be administered at intervals of time, or as a continuous release dosage form. Dosage forms or compositions may optionally comprise Compound I in the range of 0.005% to 100% (weight/weight) with the balance comprising additional substances such as those described herein. For oral administration, a pharmaceutically acceptable composition may optionally comprise any one or more commonly employed excipients, such as, for example pharmaceutical grades of mannitol, lactose, starch, magnesium stearate, talcum, cellulose derivatives, sodium crosscarmellose, glucose, sucrose, magnesium carbonate, sodium saccharin, talcum. Such compositions include solutions, suspensions, tablets, capsules, powders, dry powders for inhalers and sustained release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparing these formulations are known to those skilled in the art. The compositions may optionally contain 0.01%-100% (weight/weight) of Compound I, optionally 0.1-95%, and optionally 1-95%. Compositions according to the present invention are optionally provided for administration to humans and animals in unit dosage forms, such as tablets, capsules, pills, powders, dry powders for inhalers, granules, sterile parenteral solutions or suspensions, and oral solutions or suspensions, and oil-water emulsions containing suitable quantities of the compounds, particularly the pharmaceutically acceptable salts, preferably the sodium salts, thereof. The pharmaceutically therapeutically active compounds and derivatives thereof are typically formulated and administered in unit-dosage forms or multiple-dosage forms. Unit-dose forms, as used herein, refers to physically discrete units suitable for human and animal subjects and packaged individually as is known in the art. Each unit-dose contains a predetermined quantity of the therapeutically active compound sufficient to produce the desired therapeutic effect, in association with the required pharmaceutical carrier, vehicle or diluent. Examples of unit-dose forms include ampoules and syringes individually packaged tablet or capsule. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pint or gallons. Hence, multiple dose form is a multiple of unit-doses that are not segregated in packaging. Dosage forms or compositions may optionally comprise Compound I in the range of 0.005% to 100% (weight/weight) with the balance comprising additional substances such as those described herein. For oral administration, a pharmaceutically acceptable composition may optionally comprise any one or more commonly employed excipients, such as, for example pharmaceutical grades of mannitol, lactose, starch, magnesium stearate, talcum, cellulose derivatives, sodium crosscarmellose, glucose, sucrose, magnesium carbonate, sodium saccharin, talcum. Such compositions include solutions, suspensions, tablets, capsules, powders, dry powders for inhalers and sustained release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparing these formulations are known to those skilled in the art. The compositions may optionally contain 0.01%-100% (weight/weight) of Compound I, optionally 0.1-95%, and optionally 1-95%.

In one embodiment, the pharmaceutical composition is a pill or capsule adapted for oral administration. In another embodiment, the pharmaceutical composition is in an oral dosage form selected from the group consisting of pills, tablets, capsules, emulsions, suspensions, microsuspensions, wafers, sprinkles, chewing gum, powders, lyophilized powders, granules, and troches. In still another embodiment, the pharmaceutical composition is in a parenteral dosage

form selected from the group consisting of suspensions, microsuspensions, emulsions, solid forms suitable for suspension or emulsification prior to injection, and implantable devices. In yet another embodiment, the pharmaceutical composition is adapted for topical or transdermal administration. In a further embodiment, the pharmaceutical composition is in a topical or transdermal dosage form selected from the group consisting of suspensions, microsuspensions, emulsions, creams, gels, ointments, lotions, tinctures, pastes, powders, foams, aerosols, irrigations, sprays, suppositories, bandages, and dermal patches. In still a further embodiment, the pharmaceutical composition is in a pulmonary dosage form selected from the group consisting of powders, aerosols, suspensions, microsuspensions, and emulsions.

**A. Formulations for oral administration**

Oral pharmaceutical dosage forms may be as a solid, gel or liquid where Compound I is retained in one of the polymorphic forms. Examples of solid dosage forms include, but are not limited to tablets, capsules, granules, and bulk powders. More specific examples of oral tablets include compressed, chewable lozenges and tablets that may be enteric-coated, sugar-coated or film-coated. Examples of capsules include hard or soft gelatin capsules. Granules and powders may be provided in non-effervescent or effervescent forms. Each may be combined with other ingredients known to those skilled in the art.

In certain embodiments, Compound I is provided as solid dosage forms, preferably capsules or tablets. The tablets, pills, capsules, troches and the like may optionally contain one or more of the following ingredients, or compounds of a similar nature: a binder; a diluent; a disintegrating agent; a lubricant; a glidant; a sweetening agent; and a flavoring agent.

Examples of binders that may be used include, but are not limited to, microcrystalline cellulose, gum tragacanth, glucose solution, acacia mucilage, gelatin solution, sucrose and starch paste.

Examples of lubricants that may be used include, but are not limited to, talc, starch, magnesium or calcium stearate, lycopodium and stearic acid.

Examples of diluents that may be used include, but are not limited to, lactose, sucrose, starch, kaolin, salt, mannitol and dicalcium phosphate.

Examples of glidants that may be used include, but are not limited to, colloidal silicon dioxide.

Examples of disintegrating agents that may be used include, but are not limited to, crosscarmellose sodium, sodium starch glycolate, alginic acid, corn starch, potato starch, bentonite, methylcellulose, agar and carboxymethylcellulose.

Examples of coloring agents that may be used include, but are not limited to, any of the approved certified water soluble FD and C dyes, mixtures thereof; and water insoluble FD and C dyes suspended on alumina hydrate.

Examples of sweetening agents that may be used include, but are not limited to, sucrose, lactose, mannitol and artificial sweetening agents such as sodium cyclamate and saccharin, and any number of spray-dried flavors.

Examples of flavoring agents that may be used include, but are not limited to, natural flavors extracted from plants such as fruits and synthetic blends of compounds that produce a pleasant sensation, such as, but not limited to peppermint and methyl salicylate.

Examples of wetting agents that may be used include, but are not limited to, propylene glycol monostearate, sorbitan monooleate, diethylene glycol monolaurate and polyoxyethylene lauryl ether.

Examples of anti-emetic coatings that may be used include, but are not limited to, fatty acids, fats, waxes, shellac, ammoniated shellac and cellulose acetate phthalates.

Examples of film coatings that may be used include, but are not limited to, hydroxyethylcellulose, sodium carboxymethylcellulose, polyethylene glycol 4000 and cellulose acetate phthalate.

If oral administration is desired, Compound I may optionally be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

When the dosage unit form is a capsule, it may optionally additionally comprise a liquid carrier such as a fatty oil. In addition, dosage unit forms may optionally additionally comprise various other materials that modify the physical form of the dosage unit, for example, coatings of sugar and other enteric agents.

Compound I may also be administered as a component of an elixir, suspension, syrup, wafer, sprinkle, chewing gum or the like. A syrup may optionally comprise, in addition to the active compounds, sucrose as a sweetening agent and certain preservatives, dyes and colorings and flavors.

It is understood that the precise dosage and duration of treatment will be a function of the location of where the composition is parenterally administered, the carrier and other variables that may be determined empirically using known testing protocols or by extrapolation from *in vivo* or *in vitro* test data. It is to be noted that concentrations and dosage values may also ultimately depend on, among other criteria known to those of skill in the art. the age, weight and condition of the patient or animal, as is known in the art. It is to be further understood that for any particular subject,

specific dosage regimens may need to be adjusted over time according to the individual need and the professional judgment of the person administering or supervising the administration of the formulations. Hence, the concentration ranges set forth herein are intended to be exemplary and are not intended to limit the scope or practice of the claimed formulations.

## EXAMPLES

### Example 1: Preparation of Compound I

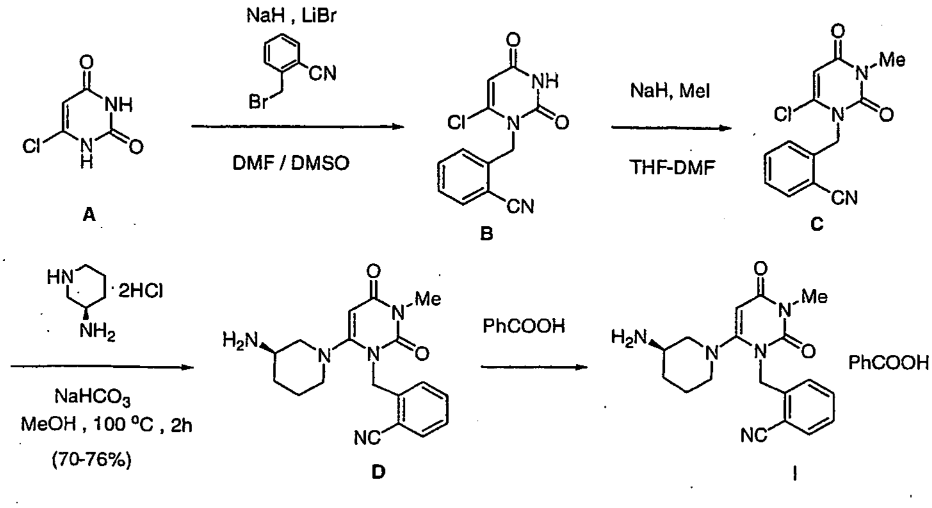


**Compound B**

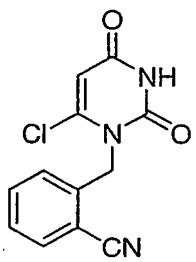

**2-(6-Chloro-2,4-dioxo-3,4-dihydro-2H-pyrimidin-1-ylmethyl)-benzonitrile** (**B**). To a solution of 6-chlorouracil (20 g, 122 mmol) in a mixture of DMF-DMSO (6:1, 600 mL) under nitrogen at 0 °C, was added sodium hydride (60%, 5.5 g, 137 mmol) in portions. After 0.5h, lithium bromide (8 g, 96 mmol) was added into the mixture and stirred for 15 min at 0 °C. A solution of α-Bromo-o-tolunitrile (25.1 g, 128 mmol) in DMF (30 mL) was added dropwise, and stirred at this temperature for 1 h, and then RT overnight. The mixture was evaporated and co-evaporated with water *in vacuo* to remove most of DMF, and then poured into ice water (1L). The precipitate was collected by filtration. The crude product was suspended in hot AcOEt-$CHCl_3$ and sonicated for 5 min, allowed to stand at 0 °C for 1h, and then filtered to give a white solid of the title compound (19 g) in 54% yield. $^1$H-NMR (400 MHz, DMSO): δ11.82 (s, 1H), 7.87 (d, 1H, J = 7.6 Hz), 7.71 (t, 1H, J = 7.6 Hz), 7.51 (t, 1H, J = 7.6 Hz), 7.37 (d, 1H, J = 8 Hz), 6.06 (s, 1H), 5.31 (s, 2H). MS (ES) [m+H] calc'd for $C_{12}H_9ClN_3O_2$, 262.0; found 262.0.

**Compound C**

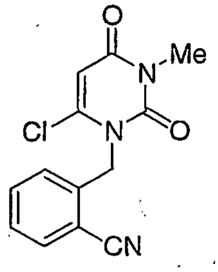

**2-(6-Chloro-3-methyl-2,4-dioxo-3,4-dihydro-2H-pyrimidin-1-ylmethyl)-benzonitrile (C).** To a cold (0 °C) solution of benzylated 6-chlorouracil 2 (10 g, 38 mmol) in DMF-THF (1:1, 300 mL) under nitrogen, was added NaH (60%, 1.6 g, 39.9 mmol) in portions, followed by adding LiBr (2g). The mixture was stirred at r.t for 20 min. After adding iodomethane (5.4 mL, 76 mmol), the flask was sealed and stirred at this temperature for 10 min, rt for 2h, and 35 °C overnight, and then concentrated in *vacuo.* The residue was dissolved in $CHCl_3$ and washed with water and brine, dried ($Na_2SO_4$), and filtered then concentrated *in vacuo.* The crude product was crystallized from THF-Hexanes to give 7.6 g (72%) of the title compound 3. $^1H$ NMR (400 MHz, DMSO): δ 7.87 (d, 1H, J = 7.6 Hz), 7.70 (t, 1H, J = 7.6 Hz), 7.51 (t, 1H, J = 7.6 Hz), 7.40 (d, 1H, J = 8 Hz), 6.21 (s, 1H), 5.38 (s, 2H), 3.28 (s, 3H). MS (ES) [m+H] calc'd for $C_{13}H_{11}ClN_3O_2$, 276.1; found 276.1.

**Compound D**

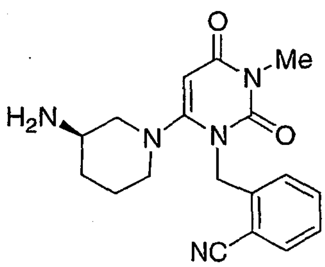

2-{6-[3(R)-Amino-piperidin-1-yl]-3-methyl-2,4-dioxo-3,4-dihydro-2H-pyrimidin-1-ylmethyl}-benzonitrile (D). 2-(6-Chloro-3-methyl-2,4-dioxo-3,4-dihydro-2-H-pyrimidin-1-ylmethyl)-benzonitrile (330 mg, 1.08 mmol), (R)-3-amino-piperidine dihydrochloride (246 mg, 1.4 mmol) and sodium bicarbonate (500mg, 5.4 mmol) were stirred with 200 mg activated molecular sieves (4A) in dry MeOH (5 mL) at 100 °C for 2 h. The reaction was filtered through Celite, concentrated *in vacuo,* and then diluted with $CHCl_3$, and washed with water. The water phase was extracted with $CHCl_3$ and the combined organic phases were washed with water, dried ($Na_2SO_4$), and filtered. TFA (1mL) was added into the solution which was then concentrated *in vacuo.* The residue was dissolved in a small amount of MeOH, and $Et_2O$ was added to force precipitation. The mixture was allowed to stand at RT overnight. Solvents were decanted, and the solid was washed with $Et_2O$ two times to give 270 mg product as off-white powder. $^1H$-NMR (400 MHz, $CDCl_3$-$CD_3OD$ 10:1): δ 7.82 (d, 1H, J = 7.6 Hz), 7.65 (t, 1H, J = 7.6 Hz), 7.46 (t, 1H, J = 7.6 Hz), 7.23 (d, 1H, J = 8.0 Hz), 5.42 (s, 1H), 5.50-5.00 (ABq, 2H, J = 41.6, 15.2 Hz), 3.30 (m, 2H), 3.16 (s, 3H), 2.91 (m, 1H), 2.76 (m, 2H), 1.93 (m, 1H), 1.79 (m, 1H), 1.51 (m, 2H). MS (ES) [m+H] calc'd for $C_{18}H_{22}N_5O_2$, 340.2; found, 340.2.

**Compound I**

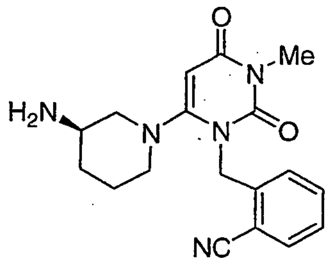

PhCOOH

The benzoic acid salt was formed by treating the benzonitrile product (**D**) with benzoic acid to form 2-[6-(3-amino-piperidin-1-yl)-3-methyl-2,4-dioxo-3,4-dihydro-2H-pyrimidin-1-ylmethyl]-benzonitrile benzoate. Preparation and isolation of the benzoate salt was performed by conventional methods for the formation of acid addition salts. $^1$H-NMR (400 MHz, $CDCl_3$-$CD_3OD$ 10:1): δ 7.82 (d, 1H, J = 7.6 Hz), 7.65 (t, 1H, J = 7.6 Hz), 7.46 (t, 1H, J = 7.6 Hz), 7.23 (d, 1H, J = 8.0 Hz), 5.42 (s, 1H), 5.50-5.00 (ABq, 2H, J = 41.6, 15.2 Hz), 3.30 (m, 2H), 3.16 (s, 3H), 2.91 (m, 1H), 2.76 (m, 2H), 1.93 (m, 1H), 1.79 (m, 1H), 1.51 (m, 2H). MS (ES) [m+H] calc'd for $C_{18}H_{22}N_5O_2$, 340.2; found, 340.2.

**Example 2: Characterization of Solubility of Compound I in Different Solvents**

The following experiments were performed in order to determine the solubility of Compound I in different solvents and solvent systems. This information was later used to identify potential crystallization conditions for Compound I.

**Materials and Reagents**

Unless otherwise stated Compound I Form A and amorphous material prepared from this sample were used as the starting materials for all crystallization experiments. Solvents and other reagents were of ACS or HPLC grade and were used as received.

**Solubility Estimates**

A weighed sample of Compound I was treated with aliquots of the test solvent at room temperature. The mixture was sonicated between aliquot additions to facilitate dissolution. Complete dissolution of the test material was determined by visual inspection. Solubilities were estimated from these experiments based on the total solvent used to provide complete dissolution. The actual solubility may be greater than those calculated because of the use of solvent aliquots that were too large or due to a slow rate of dissolution. The solubility is expressed as "less than" if dissolution did not occur during the experiment. If complete dissolution was achieved as a result of only one aliquot addition, the solubility is expressed as "greater than."

**Table 4: Solubility Estimates for Compound I Form A**

| Solvent | Sample No. | Solubility (mg/mL)[a] |
|---|---|---|
| acetone | 1924-78-02 | <3 |
| CAN | 1924-78-05 | <1 |
| $CH_2Cl_2$ | 1924-78-03 | <3 |
| DMF | 1924-77-07 | 6 |
| dioxane | 1924-78-06 | <1 |
| EtOH | 1924-77-02 | <3 |
| EtOAc | 1924-78-01 | <3 |
| IPA | 1924-77-03 | <1 |
| MeOH | 1924-77-01 | 13 |
| MEK | 1924-77-05 | 3 |
| MIBK | 1924-77-06 | <3 |

(continued)

| Solvent | Sample No. | Solubility (mg/mL)[a] |
|---|---|---|
| THF | 1924-78-04 | <1 |
| water | 1924-77-04 | 8 |
| a. Approximate solubilities rounded to nearest whole number. | | |

Form A polymorph was found to be soluble in water (8 mg/mL), methanol (13 mg/mL), dimethylformide (6 mg/mL), methyl ethyl ketone (3 mg/mL) and acetone, dichloromethane, ethanol, ethyl acetate and methyl isobutyl ketone, all at < 3 mg/mL). Thermal analysis indicates that this solid phase is thermally stable above 172 °C. DSC analysis and melting point determinations determined that Form A melts at around 172 °C. Moisture sorption/desorption analysis of form A demonstrates that this polymorph is a variable hydrate.

**Table 5.** Compound I Polymorph Screen - Solution-Based Experiments

| Solvent | Conditions[a] | Sample No. | Result |
|---|---|---|---|
| - | milled form A for 15 min/ 30Hz at RT | 1966-04-01 | disordered form A |
| - | milled form A 30 min/30Hz at RT | 1966-04-02 | disordered form A |
| | | | disordered form A[c] |
| water | wet milled form A at 30Hz for 20 mins | 2012-22-01 | form A |
| | | | form A |
| | wet milled amorphous at 30Hz for 5 mins | 1994-89-02 | form A |
| - | freeze mill 3x2min @10Hz | 1966-35-01 | disordered form A |
| acetone | FE | 1924-73-02 | form A |
| | SE | 1924-73-07 | disordered form A |
| | SC(45°C) | 1924-69-06[d] | - |
| | | 1924-94-02[d] | - |
| | SE of 1924-94-02 | 1966-16-04[d] | - |
| | RE (40°C) | 1924-83-02 | - |
| | RT slurry | 1924-97-05 | form A |
| ACN | FE | 1924-71-09 | - |
| | SE | 1966-14-03 | - |
| ACN | SC(60°C) | 1924-67-05 | form A |
| | RT slurry | 1924-97-09 | form A |
| | 60°C slurry | 1924-98-03 | form A |
| butanol | FE | 1924-71-01 | - |
| | SE | 1924-74-03 | form A |
| | SC(60°C) | 1924-66-02 | - |
| | | 1924-93-03 | - |
| | | 1966-16-02 | - |
| | RT slurry | 1924-97-07 | form A |
| | 60°C slurry | 1924-98-02 | form A |

(continued)

| Solvent | Conditions[a] | Sample No. | Result |
|---|---|---|---|
| $CH_2Cl_2$ | FE | 1924-73-01 | - |
| | SE | 1924-73-06 | - |
| | | 1966-24-01 | - |
| | SC(45°C) | 1924-69-05[c] | - |
| | | 1924-94-01[c] | - |
| | RE (40°C) | 1924-83-03 | - |
| | RT slurry | 1924-97-04 | - |
| DMF | FE | 1924-73-05 | - |
| | 1924-73-05 in 40°C oven | 1966-18-01 | - |
| | CE | 1966-29-01 | - |
| | | 1966-29-02 | - |
| | | 1966-29-03 | - |
| | SC(60°C) | 1924-69-01[c] | - |
| | | 1924-93-04[c] | - |
| | | 1966-16-03[c] | - |
| DMSO | FE | 1966-72-04 | form A |
| dioxane | FE | 1924-71-08 | - |
| | SE | 1966-31-01 | - |
| | CE | 1966-29-04 | - |
| | | 1966-29-05 | - |
| | SC(60°C) | 1924-67-03 | form A |
| | RT slurry | 1924-97-03 | form A |
| EtOH | FE | 1924-73-04 | form A |
| | SE | 1924-73-09 | form A |
| | | 1966-15-01[c] | - |
| | CE | 1966-30-01 | - |
| | | 1966-30-02 | - |
| | | 1966-30-03 | - |
| | SC(60°C) | 1924-69-03[c] | - |
| | | 1924-93-05[c] | form A |
| | RT slurry | 1924-97-08 | form A |
| EtOH-IPA | RE, evaporation | 1966-71-02 | form A |
| EtOH-water | SC(60°C) | 1966-93-01 | - |
| | SC (80°C) | 1966-61-01 | - |
| | | 1966-75-02 | - |
| | | 1994-25-01 | form A |

(continued)

| Solvent | Conditions[a] | Sample No. | Result |
|---|---|---|---|
| EtOAc | FE | 1924-71-04 | form A |
| | SE | 1924-74-05 | disordered forum A |
| | | 1966-14-04 | - |
| EtOAc | SC(60°C) | 1924-67-06 | - |
| | RT slurry | 1924-97-11 | form A |
| | 60°C slurry | 1924-98-04 | form A |
| | VS | 1966-79-02 | - |
| heptane | FE | 1924-71-07 | - |
| | FE, 60°C (1924-93-02) | 1966-16-01 | - |
| | SC(60°C) | 1924-66-01 | - |
| | | 1924-93-02 | - |
| | RT slurry | 1924-97-06 | form A |
| IPA | FE | 1924-71-11 | - |
| | | 1924-92-02[c] | - |
| | SE | 1924-74-04 | form A |
| | SC(60°C) | 1924-69-02[c] | form A |
| | RT slurry | 1924-97-02 | form A |
| | 60°C slurry | 1924-98-01 | form A |
| | VS | 1966-79-01 | - |
| IPOAc | FE | 1966-50-01 | - |
| IPOAc | SC(60°C) | 1966-50-02 | - |
| | SC(80°C) | 1994-26-02 | form A |

(continued)

| Solvent | Conditions[a] | Sample No. | Result |
|---|---|---|---|
| MeOH | FE | 1924-73-03 | - |
| | | 1924-93-01[c] | - |
| | | 1994-10-01 | - |
| | | 1994-10-02 | - |
| | | 1994-10-03 | - |
| | | 1994-10-04 | - |
| | SE | 1924-73-08 | form A |
| | CE | 1966-29-06 | - |
| | | 1966-29-07 | - |
| | SC(60°C) | 1924-69-04[c] | form A |
| | 60°C slurry | 1924-98-05 | form A |
| | RE | 1924-83-01 | - |
| | | 1966-09-01 | form A<br>endotherms: 187,211°C |
| | | 1966-73-01 | - |
| | | 1966-73-02 | - |
| | | 1966-73-03 | - |
| | | 1994-07-01 | amorphous |
| | RE, vac. | 1966-76-01 | form A + amorphous |
| | RE at RT | 1994-89-01 | amorphous |
| MEK | FE | 1924-71-10 | - |
| | | 1924-92-01 | - |
| | SE | 1924-74-01 | - |
| | SC(60°C) | 1924-67-07 | form A |
| MIBK | FE | 1924-71-05 | form A |
| | SE | 1966-14-02 | - |
| | CE | 1966-29-08 | - |
| | | 1966-29-09 | - |
| | | 1966-29-10 | - |
| | SC(60°C) | 1924-67-02 | - |
| $NO_2Me$ | FE | 1966-72-03 | - |
| TFE | VS | 1966-79-03 | - |
| | FE | 1966-72-01 | - |
| | SE | 1966-72-02 | - |
| | | 1994-31-01 | - |
| | RE, 40°C oven | 1994-02-01 | form A |

(continued)

| Solvent | Conditions[a] | Sample No. | Result |
|---|---|---|---|
| THF | FE | 1924-71-02 | - |
| | SE | 1924-73-10 | - |
| | SC(60°C) | 1924-67-04 | form A |
| | RT slurry | 1924-97-01 | form A |
| toluene | FE | 1924-71-03 | - |
| | SE | 1966-14-01 | - |
| | SC(60°C) | 1924-66-03 | - |
| | RT slurry | 1924-97-10 | form A |
| water | FE | 1924-71-06 | amorphous |
| | SE | 1924-74-02 | form A |
| | SC(60°C) | 1924-67-01 | - |
| | SC(80°C) | 1966-64-01 | - |
| water | RE | 1966-75-01 | form A |
| | RE | 1966-44-01 | form A |
| | lyophilization | 1994-12-01 | amorphous |
| | | | amorphous |
| | | 2035-15-01 | amorphous |

a. FE = fast evaporation, SE = slow evaporation, SC = slow cool, RT = room temperature, VS = vapor stress, RE = rotary evaporator.
b. B = birefringence, E = extinction. Observations made visually or using polarized light microscopy. c. Samples considered non-GMP.
§ = non-GMP observations.

## Sample Preparation

### General Methods

**Slow Evaporation (SE):** Form A was added to solvents of interest. Sonication was used to aid in dissolution. Once the mixture dissolved, as judged by visual inspection, the solution was passed through 0.2-μm nylon filter into a clean vial, covered with aluminum foil and perforated. The solution was allowed to evaporate under ambient conditions.

**Fast Evaporation (FE):** Samples were prepared according to the method for slow evaporation, except that solutions were left uncovered to evaporate.

**Slow Cool (SC):** Concentrated solutions of Form A were prepared in various solvents at ambient or at elevated temperature. The concentrated solutions were filtered through 0.2-μm nylon filters into clean vials. The solutions were allowed to slowly cool to room temperature. Further cooling to sub-ambient temperatures was achieved by placing samples in a refrigerator or freezer.

**Fast Cool (FC):** Concentrated solutions of Form A were prepared in various solvents at elevated temperatures. The concentrated solutions were filtered through 0.2-μm nylon filters into clean vials. The solutions were removed from the heat source and placed at room temperature. Further cooling to sub-ambient temperatures was achieved by placing samples in a refrigerator or freezer.

**Rotary Evaporation (RE):** Concentrated solutions of Form A were prepared in various test solvents. Solutions were passed through 0.2-μm nylon filters into clean containers, and samples were stripped to dryness using a Büchi R-114 rotavapor. Samples were immersed and rotated in a water bath at 30 or 40 °C set point during evaporation.

**Crash Precipitation (CP):** A concentrated solution of Form A was prepared in different solvents at elevated temperature. Solutions were passed through 0.2-μm nylon filters into clean vials. Antisolvent was then added to the sample solutions. Precipitates that formed were collected by vacuum filtration.

**Slurries:** Saturated solutions of Form A containing excess solid were prepared in various solvents. The samples

were placed on a shaker block and agitated at a set temperature for a period of time. Solids were later collected for analysis by decanting the solutions or by vacuum filtration.

**Vapor Diffusions (VD):** Form A was dissolved in test solvents. These solutions were passed through a 0.2-μm filter into small vials. The small vials were placed, uncovered, into larger vials containing a miscible antisolvent. The large vials were capped, sealed with Parafilm® and kept at ambient conditions. Solids that formed were isolated and analyzed.

**Vapor Stress (VS) Experiments:** Form A was dispensed into a small vial, which was placed, uncapped, in a larger vial containing a diffusing solvent. The larger vial was sealed and stored at ambient temperature. Samples were also stressed in ovens at elevated temperatures and under different relative humidity conditions.

**Capillary Evaporations (CE):** Solutions of Form A were filled into glass capillaries by centrifugation. The capillary samples were then allowed to evaporate.

**Crash Cools (CC):** Samples of Form A in different solvents were prepared and passed through 0.2- μm nylon filters into clean vials. The vials containing solutions were then rapidly cooled by submersion in a dry ice/acetone bath for several seconds. Solids that precipitate were collected by filtration and dried.

**Milling Experiments:** A small amount of Form A was placed in a grinding holder with milling ball and the holder capped. The sample was then ground in a mixer miller at a frequency of 30Hz for a measured time interval. The solids were examined using optical microscopy. Several samples were wet-milled by adding sufficient water to the sample to dampen it before milling.

**Cryogrinding Experiments:** A small amount of Form A was ground at a frequency of 10 Hz for a total of 6 minutes using a Spex Centriprep 6750 freezer miller filled with liquid nitrogen. The sample was allowed to cool for one minute after every two minute grinding cycle. The sample was removed from the mill and allowed to equilibrate to room temperature in a large desiccator.

**<u>Example 3:</u> X-Ray Powder Diffraction**

X-ray powder diffraction analyses were performed using a Shimadzu XRD-6000 X-ray powder diffractometer using Cu Kα radiation. The instrument is equipped with a long fine focus X-ray tube. The tube voltage and amperage were set to 40 kV and 40 mA, respectively. The divergence and scattering slits were set at 1° ° and the receiving slit was set at 0.15 mm. Diffracted radiation was detected by a NaI scintillation detector. A θ-2θ continuous scan at 3 °/min (0.4 sec/0.02° step) from 2.5 to 40 °2θ was used. A silicon standard was analyzed to check the instrument alignment. Data were collected and analyzed using XRD-6000 v. 4.1. Samples were prepared for analysis by placing them in an aluminum holder with silicon insert.

X-ray powder diffraction (XRPD) analyses were also performed using an Inel XRG-3000 diffractometer equipped with a CPS (Curved Position Sensitive) detector with a 2θ range of 120°. Real time data were collected using Cu-Karadiation starting at approximately 4 °2θ at a resolution of 0.03 °2θ. The tube voltage and amperage were set to 40 kV and 30 mA, respectively. The monochromator slit was set at 5 mm by 80 μm. The pattern is displayed from 2.5-40 °2θ Samples were prepared for analysis by packing them into thin-walled glass capillaries. Each capillary was mounted onto a goniometer head that is motorized to permit spinning of the capillary during data acquisition. The samples were analyzed for 5 min. Instrument calibration was performed using a silicon reference standard.

Data Collection: A colorless plate of $C_{18}H_{22}N_5O_2,C_7H_5O_2$ having approximate dimensions of 0.50 x 0.35 x 0.28 mm was mounted on a glass fiber in a random orientation. Preliminary examination and data collection were performed Mo $K_\alpha$ radiation ($\lambda$ = 0.71073Å) on a Nonius KappaCCD equipped with a graphite crystal, incident beam monochromator. Cell constants for data collection were obtained from least-squares refinement, using the setting angles of 13398 reflections in the range 2 < θ < 27°. The refined mosaicity from DENZO/SCALEPACK was 0.41° indicating good crystal quality. The space group was determined by the program XPREP. From the systematic presences of: *h*00 *h*=2n, 0*k*0 *k*=2n, 00*l* *l*=2n and from subsequent least-squares refinement, the space group was determined to be $P2_12_12_1$ (no. 19). The data were collected at a temperature of 423 K. Data were collected to a maximum 2θ of 55.1°.

Data Reduction: A total of 13398 reflections were collected, of which 4589 were unique. Frames were integrated with DENZO-SMN. Lorentz and polarization corrections were applied to the data. The linear absorption coefficient is 0.9 /cm for Mo $K_\alpha$ radiation. An empirical absorption correction using SCALEPACK was applied. Transmission coefficients ranged from 0.943 to 0.976. A secondary extinction correction was applied. Intensities of equivalent reflections were averaged. The agreement factor for the averaging was 10.2 % based on intensity.

Structure Solution and Refinement: The structure was solved by direct methods using SIR2002. The remaining atoms were located in succeeding difference Fourier syntheses. Hydrogen atoms were included in the refinement but restrained to ride on the atom to which they are bonded. The structure was refined in full-matrix least-squares by minimizing the function:

$$\sum w\left(|F_o|^2 - |F_c|^2\right)^2$$

The weight w is defined as $1/[\sigma^2(F_o^2)+(0.1225P)^2]$ where $P=(F_o^2 + 2F_c^2)/3$.

Scattering factors were taken from the "International Tables for Crystallography". Of the 4589 reflections were used in the refinements, only the 2414 reflections with $F_o^2>2\sigma(F_o^2)$ were used in, calculating *R*. The final cycle of refinement included 321 variable parameters and converged (largest parameter shift was <0.01 times its estimated standard deviation) with unweighted and weighted agreement factors of:

$$R = \sum|F_o - F_c| / \sum F_o = 0.068$$

$$R_w = \sqrt{\left(\sum w\left(F_o^2 - F_c^2\right)^2 / \sum w\left(F_o^2\right)^2\right)} = 0.150$$

The standard deviation of an observation of unit weight was 1.06. The highest peak in the final difference Fourier had a height of 0.34 e/A$^3$. The minimum negative peak had a height of -0.45 e/A$^3$. The factor for the determination of the absolute structure refined to -1.00. Refinement was performed on a LINUX PC using SHELX-97. Crystallographic drawings were done using programs ORTEP.

Computation of Disorder and Amorphous Content of Milled Samples: The weight percent crystallinity of milled samples was determined by calculation using two software packages. Shimadzu percent crystallinity module, which is part of the Shimadzu XRD-6000 software package, was used for samples with significant amorphous content. In-house software was used for largely crystalline samples, as the Shimadzu software was less accurate at low amorphous concentrations. For the in-house software, X-ray powder data was first smoothed and then a series of digital filters is applied to separate the data into three components: crystalline, amorphous and disordered. A background correction was also applied. The percent amorphous content was then calculated by determining the ratio of amorphous to the sum total of all three components. Both methods were run under non-GMP conditions and provide approximate values only.

Milling Studies: Dry milling Form A at ambient and liquid nitrogen temperatures generated disordered Form A. Peak broadening, an offset baseline and reduced signal intensity were characteristic of the XRPD patterns of dry milled samples. Wet milling Form A and amorphous material with water produced crystalline Form A solids. Four milled samples were analyzed by XRPD. All patterns matched Form A, without visible amorphous content.

Calculation of Amorphous Content of a Milled Sample: XRPD patterns of milled samples of Compound I Forma A exhibited peak broadening as well as an amorphous halo. This suggested that two processes were contributing to the loss of crystallinity: crystallite size reduction causing the peak broadening and amorphous formation causing the offset baseline. A dry milled sample 1966-04-02 (milled for 30 minutes) was chosen for the study and the XRPD pattern of Form A (lot no. QZ-656-17(1)) used as a reference standard of Form A. The weight percent of amorphous material of the dry milled sample 1966-04-02 was estimated to be around 58 % by calculation. Lot 635-181-1 contained around 13 % amorphous content by weight. Values determined by computation are approximate, particularly at low amorphous content.

## <u>Example 4:</u> Differential Scanning Calorimetry (DSC)

Differential scanning calorimetry was performed using a TA Instruments differential scanning calorimeter 2920. The sample was placed into an aluminum DSC pan, and the weight accurately recorded. The pan was covered with a lid and then crimped. The sample cell was equilibrated at ambient temperature and heated under a nitrogen purge at a rate of 10 °C/min, up to a final temperature of 350 °C. Indium metal was used as the calibration standard. Reported temperatures are at the transition maxima.

For studies of the glass transition temperature ($T_g$) of the amorphous material, the sample cell was equilibrated at room temperature, then heated under nitrogen at a rate of 10 °C/min, up to 100 °C. The sample cell was then cooled to -40 °C before being heated again at a rate of 10 °C/min up to a final temperature of 350 °C. The *Tg* is reported from the inflection point of the transition.

Cyclic DSC experiments were carried out by placing accurately weighed samples in uncrimped pans. Samples were heated under nitrogen at a rate of 10 °C/min to either 150 or 180 °C subsequently cooled to -40 °C. This procedure was repeated twice before the sample was heated to 250 °C.

**Example 5: Thermogravimetric Analysis (TGA)**

Thermogravimetric analyses were performed using a TA Instruments 2950 thermogravimetric analyzer. Each sample was placed in an aluminum sample pan and inserted into the TG furnace. The furnace was first equilibrated at ambient temperature, then heated under nitrogen at a rate of 10 °C/min, up to a final temperature of 350 °C. Nickel and Alumel™ were used as the calibration standards.

**Example 6: Hot stage Microscopy (HSM)**

Hot stage microscopy was performed using a Linkam hot stage mounted on a Leica DM LP microscope. Samples were observed using a 20× objective with a lambda plate with crossed polarizers. Samples were sandwiched between two coverslips and visually observed as the stage was heated. Images were captured using a SPOT Insight™ color digital camera with SPOT Software v. 3.5.8. The hot stage was calibrated using sulfapyridine and vanillin USP melting point standards.

**Example 7: Fourier Transform Infrared Spectroscopy (FT-IR)**

Infrared spectra were acquired on a Magna-IR 860® Fourier transform infrared (FT-IR) spectrophotometer (Thermo Nicolet) equipped with an Ever-Glo mid/far IR source, a potassium bromide (KBr) beamsplitter, and a deuterated triglycine sulfate (DTGS) detector. An ATR Thunderdome accessory with non-concave tip was used for sampling. Sample preparation consisted of placing the sample on a germanium crystal and pressing the material against the crystal using a plunger. Each spectrum represents 256 co-added scans collected at a spectral resolution of 4 $cm^{-1}$. An air background data set was acquired. A Log 1/*R* (*R* = reflectance) spectrum was acquired by taking a ratio of these two data sets against each other. Wavelength calibration was performed using polystyrene.

Infrared spectra were also acquired using a diffuse reflectance accessory (the Collector™, Thermo Spectra-Tech). Sample preparation consisted of placing the sample into a 13-mm diameter cup. A background data set was acquired with an alignment mirror in place. A Log 1/*R* (*R* = reflectance) spectrum was acquired by taking a ratio of these two data sets against each other. Wavelength calibration was performed using polystyrene.

**Example 8: Raman Spectroscopy**

FT-Raman spectra were acquired on an FT-Raman 960 spectrometer (Thermo Nicolet). This spectrometer uses an excitation wavelength of 1064 nm. Approximately 0.6-0.8 W of $Nd:YVo_4$ laser power was used to irradiate the samples. The Raman spectra were measured with an indium gallium arsenide (InGaAs) detector. The samples were prepared for analysis by placing the material in a glass capillary or NMR tube. A total of 256 sample scans were collected from 400 - 3600 $cm^{-1}$ at a spectral resolution of 4 $cm^{-1}$, using Happ-Genzel apodization. Wavelength calibration was performed using sulfur and cyclohexane.

**Example 9: NMR Spectroscopy**

Solution $^1H$ NMR spectra can be acquired at ambient temperature on a Bruker Instruments AM-250 spectrometer at a magnetic field strength of 5.87 Tesla ($^1H$ Larmor frequency = 250 MHz). The samples can be dissolved in NMR-grade DMSO-$d_6$. Spectra can be acquired with a $^1H$ pulse width of 8.5 μs (90°), a 2.5 second acquisition time, a 5.0 second delay between scans, a spectral width of 6400.0 Hz with 32K data points, and 32 co-added scans. Each free induction decay (FID) can be processed with GRAMS/32 AI software v. 6.00 using a Fourier number equal to twice the number of acquired points [or a larger multiple if zero filling is used] with an exponential line broadening factor of 0.2 Hz to improve sensitivity. Peak tables can be generated by the GRAMS software peak picking algorithm. For these spectra the residual peak from incompletely deuterated DMSO-$d_6$ is located at approximately 2.50 ppm.

**Example 10: Moisture Sorption/Desorption Analyses**

Moisture sorption/desorption data were collected on a VTI SGA-100 Vapor Sorption Analyzer. Sorption and desorption data were collected over a range of 5 % to 95 % relative humidity (RH) at 10 % RH intervals under a nitrogen purge. Samples were not dried prior to analysis. Equilibrium criteria used for analysis were less than 0.0100 % weight change in 5 minutes, with a maximum equilibration time of 3 hours if the weight criterion was not met. Data were not corrected for the initial moisture content of the samples. NaCl and PVP were used as calibration standards.

**Example 11: Karl Fischer Water Analysis**

Karl Fischer (titrimetric) water analysis can be performed according to U.S. Pharmacopoeia, vol. 24, method 921, U.S.P. Pharmacopeial Convention, Inc, Rockville, Md. The polymorph can be tested for water content by Karl Fischer titration using a coulometer according to the published procedure and the manufacturer's coulometer instructions.

## Claims

1. A method for preparing Compound I having the formula

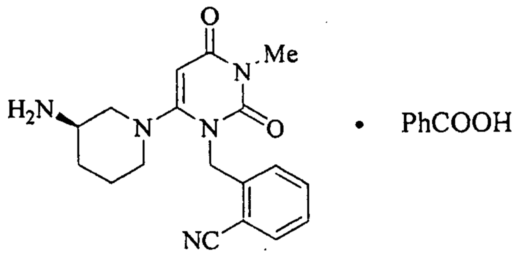

wherein at least a portion of Compound I is present in polymorph Form A **characterized by** one or more physical properties selected from the group consisting of:

an X-ray powder diffraction pattern with salient features being major diffraction lines as follows:

| **Position (°2θ)** | 9.44 | 10.84 | 17.82 | 18.75 | 25.87 | 28.52 |
|---|---|---|---|---|---|---|

using Cu-Kα radiation;
an IR spectrum comprising absorption peaks at 1212, 1365, 1447, 1613 and 1697 $cm^{-1}$;
an FT-Raman spectrum comprising peaks at 1065, 1103, 1235, 1288, 1337, 1365, 1624, 1689, 2883, 2983 and 3026 $cm^{-1}$; and
a differential scanning calorimetry spectrum having an endotherm range at about 173 °C to about 195 °C,
the method comprising:

(a) crystallization from any of the following solvent systems comprising (i) acetone, (ii) acetonitrile; (iii) butanol, (iv) dimethylsulfoxide; (v) dioxane; (vi) ethanol; (vii) ethanol and isopropyl alcohol; (viii) ethanol and water; (ix) ethyl acetate; (x) heptane; (xi) isopropanol; (xii) isopropyl acetate; (xiii) methanol; (xiv) methyl ethyl ketone; (xv) methyl isobutyl ketone; (xvi) 2,2,2-trifluoroethanol; (xvii) tetrahydrofuran; (xviii) toluene; (xix) water; and (xx) ethanol and heptane; or
(b) by the conversion of an amorphous Form 1 by stressing the amorphous Form 1 with heat, high relative humidity or organic vapors, or by wet milling of amorphous Form 1 with water.

2. A composition comprising Compound I having the formula

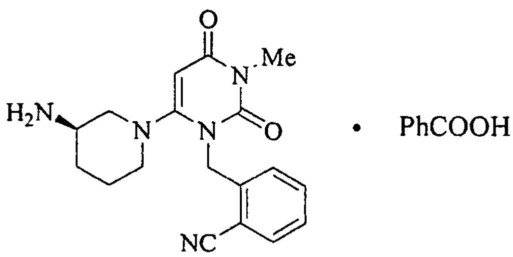

wherein at least a portion of Compound I is present in the composition as amorphous Form 1 **characterized by** one or more physical properties selected from the group consisting of:

an X-ray powder diffraction pattern that shows a broad halo with no specific peaks present using Cu-Kα radiation;
an IR spectrum comprising absorption peaks at 1703, 1599, 1119, 868 and 809 $cm^{-1}$;
an FT-Raman spectrum comprising peaks at 805, 1280 and 1703 $cm^{-1}$; and
a differential scanning calorimetry spectrum having a *Tg* = 70 °C (onset), an exotherm at 132 °C, and an endotherm onset at 183 °C.

**3.** The composition according to claim 2 wherein at least a portion of Compound I is present in the composition as amorphous Form 1 **characterized by** one or more physical properties selected from the group consisting of:

an X-ray powder diffraction pattern that shows a broad halo with no specific peaks present using Cu-Kα radiation;
an IR spectrum comprising absorption peaks at 3062, 2949, 2861, 2571, 2225, 2136, 1703, 1652, 1599, 1541, 1440, 1375, 1286, 1228, 1172, 1134, 1119, 1084, 1068, 1024, 948, 868, 833, and 809 $cm^{-1}$;
an FT-Raman spectrum comprising peaks at 805, 834, 904, 1002, 1024, 1045, 1134, 1168, 1205, 1280, 1386, 1443, 1578, 1600, 1654, 1703, 2225, 2864, 2958 and 3065 $cm^{-1}$; and
a differential scanning calorimetry spectrum having a *Tg* = 70 °C (onset), an exotherm at 132 °C, and an endotherm onset at 183 °C.

**4.** A pharmaceutical composition comprising:

Compound I having the formula

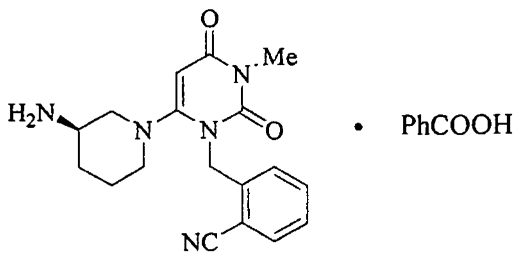

wherein at least a portion of Compound I is present in the pharmaceutical composition as amorphous Form 1 **characterized by** one or more physical properties selected from the group consisting of:

an X-ray powder diffraction pattern that shows a broad halo with no specific peaks present using Cu-Kα radiation;
an IR spectrum comprising absorption peaks at 1703, 1599, 1119, 868 and 809 $cm^{-1}$
an FT-Raman spectrum comprising peaks at 805, 1280 and 1703 $cm^{-1}$; and
a differential scanning calorimetry spectrum having a *Tg* = 70 °C (onset), an exotherm at 132 °C, and an endotherm onset at 183 °C; and
one or more pharmaceutical carriers.

**5.** The pharmaceutical composition according to claim 4, wherein at least a portion of Compound I is present in the pharmaceutical composition as amorphous Form 1 **characterized by** one or more physical properties selected from the group consisting of:

an X-ray powder diffraction pattern that shows a broad halo with no specific peaks present using Cu-Kα radiation;
an IR spectrum comprising absorption peaks at 3062, 2949, 2861, 2571, 2225, 2136, 1703, 1652, 1599, 1541, 1440, 1375, 1286, 1228, 1172, 1134, 1119, 1084, 1068, 1024, 948, 868, 833, and 809 $cm^{-1}$;
an FT-Raman spectrum comprising peaks at 805, 834, 904, 1002, 1024, 1045, 1134, 1168, 1205, 1280, 1386, 1443, 1578, 1600, 1654, 1703, 2225, 2864, 2958 and 3065 $cm^{-1}$; and
a differential scanning calorimetry spectrum having a *Tg* = 70 °C (onset), an exotherm at 132 °C, and an endotherm onset at 183 °C,; and
one or more pharmaceutical carriers.

**6.** The pharmaceutical composition according to any one of claims 4 and 5, wherein the composition is a pill or capsule adapted for oral administration.

**7.** The pharmaceutical composition according to any one of claims 4 and 5, wherein the composition is in an oral dosage form selected from the group consisting of pills, tablets, capsules, emulsions, suspensions, microsuspensions, wafers, sprinkles, chewing gum, powders, lyophilized powders, granules, and troches.

**8.** The pharmaceutical composition according to any one of claims 4 and 5, wherein the composition is in a parenteral dosage form selected from the group consisting of suspensions, microsuspensions, emulsions, solid forms suitable for suspension or emulsification prior to injection, and implantable devices.

**9.** The pharmaceutical composition according to any one of claims 4 and 5, wherein the composition is adapted for topical or transdermal administration.

**10.** The pharmaceutical composition according to any one of claims 4 and 5, wherein the composition is in a topical or transdermal dosage form selected from the group consisting of suspensions, microsuspensions, emulsions, creams, gels, ointments, lotions, tinctures, pastes, powders, foams, aerosols, irrigations, sprays, suppositories, bandages, and dermal patches.

**11.** The pharmaceutical composition according to any one of claims 4 and 5, wherein the composition is in a pulmonary dosage form selected from the group consisting of powders, aerosols, suspensions, microsuspensions, and emulsions.

**12.** The composition according to any one of claims 2-11, wherein greater than 1% of Compound I (by weight) is present in the composition as amorphous Form 1.

**13.** The composition according to any one of claims 2-11, wherein greater than 5% of Compound I (by weight) is present in the composition as amorphous Form 1.

**14.** The composition according to any one of claims 2-11, wherein greater than 50% of Compound I (by weight) is present in the composition as amorphous Form 1.

**15.** The composition according to any one of claims 2-11, wherein greater than 75% of Compound I (by weight) is present in the composition as amorphous Form 1.

**16.** The composition according to any one of claims 2-11, wherein greater than 90% of Compound I (by weight) is present in the composition as amorphous Form 1.

**17.** The composition according to any one of claims 2-11, wherein greater than 95% of Compound I (by weight) is present in the composition as amorphous Form 1.

**18.** The composition according to any one of claims 2-11, wherein greater than 97% of Compound I (by weight) is present in the composition as amorphous Form 1.

**19.** The composition according to any one of claims 2-11, wherein greater than 99% of Compound I (by weight) is present in the composition as amorphous Form 1.

**20.** A kit comprising:

a pharmaceutical composition comprising Compound I having the formula

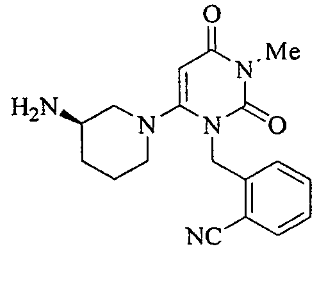 • PhCOOH

wherein at least a portion of Compound I is present in the pharmaceutical composition as amorphous Form 1 **characterized by** one or more physical properties selected from the group consisting of:

an X-ray powder diffraction pattern that shows a broad halo with no specific peaks present using Cu-K$\alpha$ radiation;
an IR spectrum comprising absorption peaks at 1703, 1599, 1119, 868 and 809 $cm^{-1}$;
an FT-Raman spectrum comprising peaks at 805, 1280 and 1703 $cm^{-1}$; and
a differential scanning calorimetry spectrum having a *Tg* = 70 °C (onset), an exotherm at 132 °C, and an endotherm onset at 183 °C; and
instructions which comprise one or more forms of information selected from the group consisting of indicating a disease state for which the composition is to be administered, storage information for the composition, dosing information and instructions regarding how to administer the composition.

**21.** The kit according to claim 20, wherein at least a portion of Compound I is present in the pharmaceutical composition as amorphous Form 1 **characterized by** one or more physical properties selected from the group consisting of:

an X-ray powder diffraction pattern that shows a broad halo with no specific peaks present using Cu-K$\alpha$ radiation;
an IR spectrum comprising absorption peaks at 3062, 2949, 2861, 2571, 2225, 2136, 1703, 1652, 1599, 1541, 1440, 1375, 1286, 1228, 1172, 1134, 1119, 1084, 1068, 1024, 948, 868, 833, and 809 $cm^{-1}$;
an FT-Raman spectrum comprising peaks at 805, 834, 904, 1002, 1024, 1045, 1134, 1168, 1205, 1280, 1386, 1443, 1578, 1600, 1654, 1703, 2225, 2864, 2958 and 3065 $cm^{-1}$; and
a differential scanning calorimetry spectrum having a *Tg* = 70 °C (onset), an exotherm at 132 °C, and an endotherm onset at 183 °C; and
instructions which comprise one or more forms of information selected from the group consisting of indicating a disease state for which the composition is to be administered, storage information for the composition, dosing information and instructions regarding how to administer the composition.

**22.** The kit according to any one of claims 20 and 21, wherein the kit comprises the composition in a multiple dose form.

**23.** An article of manufacture comprising:

a composition according to any one of claims 2-19; and
packaging materials.

**24.** The article of manufacture according to claim 23, wherein the packaging material comprises a container for housing the composition.

**25.** The article of manufacture according to claim 24, wherein the container comprises a label indicating one or more members of the group consisting of a disease state for which the composition is to be administered, storage information, dosing information and/or instructions regarding how to administer the composition.

**26.** The article of manufacture according to claim 23, wherein the article of manufacture comprises the composition in a multiple dose form.

**27.** A composition according to any one of claims 2-19 for use as a medicament.

**28.** Use of a composition according to any one of claims 2-19 in the preparation of a medicament for treating diabetes.

**29.** Use of a composition according to any one of claims 2-19 in the preparation of a medicament for treating Type II diabetes.

**30.** Use of a composition according to any one of claims 2-19 in the preparation of a medicament for treating or preventing cancer metastases.

**31.** A composition according to any one of claims 2-19 for use in treating diabetes.

**32.** A composition according to any one of claims 2-19 for use in treating Type II diabetes.

**33.** A composition according to any one of claims 2-19 for use in treating or preventing cancer metastases.

**34.** A method for preparing Compound I having the formula

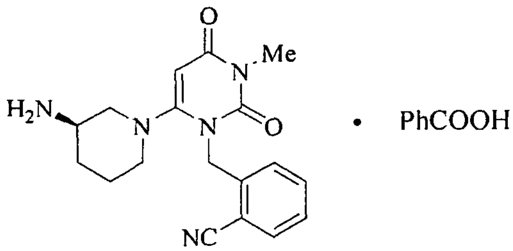

wherein at least a portion of Compound I is present in amorphous Form 1, the method selected from the group consisting of:

(a) rotoevaporation from methanol;
(b) fast evaporation from water;
(c) lyophilization from water;
(d) crystallization from ethyl acetate and hexanes; and crystallization from isopropyl acetate and hexanes, and isolating Compound I from the solvent.

## Patentansprüche

**1.** Verfahren zur Herstellung der Verbindung I, welche die Formel

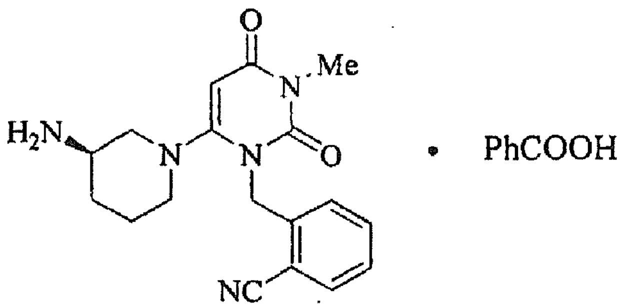

aufweist,
wobei wenigstens ein Teil der Verbindung I in der polymorphen Form A vorliegt, welche durch eine oder mehrere physikalische Eigenschaften **gekennzeichnet** ist, die ausgewählt sind aus der Gruppe bestehend aus:

einem Röntgenpulverbeugungsdiagramm unter Verwendung von Cu-Kα-Strahlung, das sich durch die folgenden Hauptbeugungslinien auszeichnet:

| **Position (°2θ)** | 9,44 | 10,84 | 17,82 | 18,75 | 25,87 | 28,52 |
|---|---|---|---|---|---|---|

einem IR-Spektrum, welches Absorptionsmaxima bei 1212, 1365, 1447, 1613 und 1697 $cm^{-1}$ umfasst,
einem FT-Raman-Spektrum, welches Maxima bei 1065, 1103, 1235, 1288, 1337, 1365, 1624, 1689, 2883, 2983 und 3026 $cm^{-1}$ umfasst, und
einem Differentialscanningkalorimetrie-Spektrum, welches bei etwa 173 °C bis etwa 195 °C einen endothermen Bereich aufweist,
wobei das Verfahren umfasst:

(a) Kristallisation aus einem der folgenden Lösungsmittelsysteme umfassend (i) Aceton, (ii) Acetonitril, (iii)

Butanol, (iv) Dimethylsulfoxid, (v) Dioxan, (vi) Ethanol, (vii) Ethanol und Isopropylalkohol, (viii) Ethanol und Wasser, (ix) Ethylacetat, (x) Heptan, (xi) Isopropanol, (xii) Isopropylacetat, (xiii) Methanol, (xiv) Methylethylketon, (xv) Methylisobutylketon, (xvi), 2,2,2-Trifluorethanol, (xvii) Tetrahydrofuran, (xviii) Toluen, (xix) Wasser, und (xx) Ethanol und Heptan, oder
(b) durch Umwandlung einer amorphen Form 1 durch Belasten der amorphen Form 1 mit Hitze, hoher relativer Feuchtigkeit oder organischen Dämpfen, oder durch Nassmahlung der amorphen Form 1 mit Wasser.

**2.** Zusammensetzung, welche die Verbindung I mit der Formel

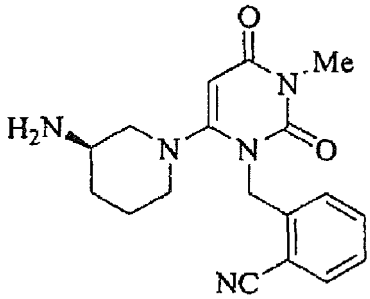

• PhCOOH

umfasst, wobei wenigstens ein Teil der Verbindung I als amorphe Form 1 in der Zusammensetzung vorliegt, **gekennzeichnet durch** eine oder mehrere physikalische Eigenschaften, die ausgewählt sind aus der Gruppe bestehend aus:

einem Röntgenpulverbeugungsdiagramm unter Verwendung von Cu-Kα-Strahlung, das ein breites Halogen zeigt, wobei keine spezifischen Maxima vorliegen,
einem IR-Spektrum, welches Absorptionsmaxima bei 1703, 1599, 1119, 868 und 809 $cm^{-1}$ umfasst,
einem FT-Raman-Spektrum, welches Maxima bei 805, 1280 und 1703 $cm^{-1}$ umfasst, und
einem Differentialscanningkalorimetrie-Spektrum mit *Tg* = 70 °C (Beginn), einem Exothermen bei 132 °C und einem endothermen Beginn bei 183 °C.

**3.** Zusammensetzung gemäß Anspruch 2, wobei wenigstens ein Teil der Verbindung I als amorphe Form 1 in der Zusammensetzung vorliegt, und durch eine oder mehrere physikalische Eigenschaften **gekennzeichnet** ist, die ausgewählt sind aus der Gruppe bestehend aus:

einem Röntgenpulverbeugungsdiagramm unter Verwendung von Cu-Kα-Strahlung, das ein breites Halogen zeigt, wobei keine spezifischen Maxima vorliegen,
einem IR-Spektrum, welches Absorptionsmaxima bei 3062, 2949, 2861, 2571, 2225, 2136, 1703, 1652, 1599, 1541, 1440, 1375, 1286, 1228, 1172, 1134, 1119, 1084, 1068, 1024, 948, 868, 833 und 809 $cm^{-1}$ umfasst,
einem FT-Raman-Spektrum, welches Maxima bei 805, 834, 904, 1002, 1024, 1045, 1134, 1168, 1205, 1280, 1386, 1443, 1578, 1600, 1654, 1703, 2225, 2864, 2958 und 3065 $cm^{-1}$ umfasst, und
einem Differentialscanningkalorimetrie-Spektrum mit *Tg* = 70 °C (Beginn), einem Exothermen bei 132 °C und einem endothermen Beginn bei 183 °C.

**4.** Pharmazeutische Zusammensetzung, die Verbindung I mit der Formel

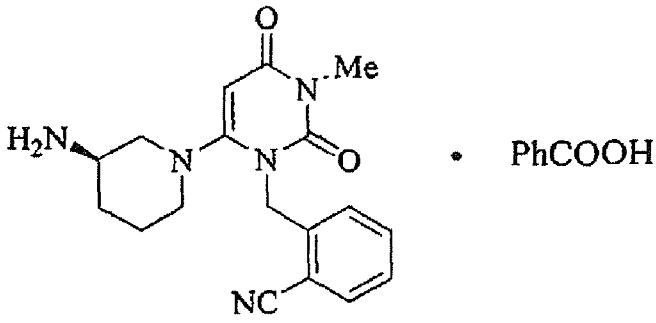

wobei wenigstens ein Teil der Verbindung I in der pharmazeutischen Zusammensetzung als amorphe Form 1 vorliegt, **gekennzeichnet durch** eine oder mehrere physikalische Eigenschaften, die ausgewählt sind aus der Gruppe bestehend aus:

einem Röntgenpulverbeugungsdiagramm unter Verwendung von Cu-Kα-Strahlung, das ein breites Halogen zeigt, wobei keine spezifischen Maxima vorliegen,
einem IR-Spektrum, welches Absorptionsmaxima bei 1703, 1599, 1119, 868 und 809 $cm^{-1}$ umfasst,
einem FT-Raman-Spektrum, welches Maxima bei 805, 1280 und 1703 $cm^{-1}$ umfasst, und
einem Differentialscanningkalorimetrie-Spektrum mit *Tg* = 70 °C (Beginn), einem Exothermen bei 132 °C und einem endothermen Beginn bei 183 °C, und
einen oder mehrere pharmazeutische Träger umfasst.

**5.** Pharmazeutische Zusammensetzung gemäß Anspruch 4, wobei wenigstens ein Teil der Verbindung I in der pharmazeutischen Zusammensetzung als amorphe Form 1 vorliegt, **gekennzeichnet durch** eine oder mehrere physikalische Eigenschaften, die ausgewählt sind aus der Gruppe bestehend aus:

einem Röntgenpulverbeugungsdiagramm unter Verwendung von Cu-Kα-Strahlung, das ein breites Halogen zeigt, wobei keine spezifischen Maxima vorliegen,
einem IR-Spektrum, welches Absorptionsmaxima bei 3062, 2949, 2861, 2571, 2225, 2136, 1703, 1652, 1599, 1541, 1440, 1375, 1286, 1228, 1172, 1134, 1119, 1084, 1068, 1024, 948, 868, 833 und 809 $cm^{-1}$ umfasst,
einem FT-Raman-Spektrum, welches Maxima bei 805, 834, 904, 1002, 1024, 1045, 1134, 1168, 1205, 1280, 1386, 1443, 1578, 1600, 1654, 1703, 2225, 2864, 2958 und 3065 $cm^{-1}$ umfasst, und
einem Differentialscanningkalorimetrie-Spektrum mit *Tg* = 70 °C (Beginn), einem Exothermen bei 132 °C und einem endothermen Beginn bei 183 °C, und
ein oder mehrere pharmazeutische Träger.

**6.** Pharmazeutische Zusammensetzung gemäß einem der Ansprüche 4 und 5, wobei die Zusammensetzung eine Pille oder Kapsel ist, die zur oralen Verabreichung geeignet ist.

**7.** Pharmazeutische Zusammensetzung gemäß einem der Ansprüche 4 und 5, wobei die Zusammensetzung in einer oralen Verabreichungsform vorliegt, ausgewählt aus der Gruppe bestehend aus Pillen, Tabletten, Kapseln, Emulsionen, Suspensionen, Mikrosuspensionen, Oblaten, Streuseln, Kaugummis, Pulvern, lyophilisierten Pulvern, Granula und Pastillen.

**8.** Pharmazeutische Zusammensetzung gemäß einem der Ansprüche 4 und 5, wobei die Zusammensetzung in parenteraler Verabreichungsform vorliegt, ausgewählt aus der Gruppe bestehend aus Suspensionen, Mikrosuspensionen, Emulsionen, festen Formen zum Suspendieren oder Emulgieren vor der Injektion, und implantierbaren Vorrichtungen.

**9.** Pharmazeutische Zusammensetzung gemäß einem der Ansprüche 4 und 5, wobei die Zusammensetzung zur topischen oder transdermalen Verabreichung geeignet ist.

**10.** Pharmazeutische Zusammensetzung gemäß einem der Ansprüche 4 und 5, wobei die Zusammensetzung in topischer oder transdermaler Verabreichungsform vorliegt, ausgewählt aus der Gruppe bestehend aus Suspensionen, Mikrosuspensionen, Emulsionen, Cremes, Gelen, Salben, Lotionen, Tinkturen, Pasten, Pudern, Schäumen, Aerosolen, Spülungen, Sprays, Zäpfchen, Bandagen und Hautpflastern.

**11.** Pharmazeutische Zusammensetzung gemäß einem der Ansprüche 4 und 5, wobei die Zusammensetzung in pulmonaler Verabreichungsform vorliegt, ausgewählt aus der Gruppe bestehend aus Pulvern, Aerosolen, Suspensionen, Mikrosuspensionen und Emulsionen.

**12.** Zusammensetzung nach einem der Ansprüche 2 bis 11, wobei mehr als 1 % der Verbindung I (nach Gewicht) als amorphe Form 1 in der Zusammensetzung vorliegt.

**13.** Zusammensetzung nach einem der Ansprüche 2 bis 11, wobei mehr als 5 % der Verbindung I (nach Gewicht) als amorphe Form 1 in der Zusammensetzung vorliegt.

**14.** Zusammensetzung nach einem der Ansprüche 2 bis 11, wobei mehr als 50 % der Verbindung I (nach Gewicht) als amorphe Form 1 in der Zusammensetzung vorliegt.

**15.** Zusammensetzung nach einem der Ansprüche 2 bis 11, wobei mehr als 75 % der Verbindung I (nach Gewicht) als amorphe Form 1 in der Zusammensetzung vorliegt.

**16.** Zusammensetzung nach einem der Ansprüche 2 bis 11, wobei mehr als 90 % der Verbindung I (nach Gewicht) als amorphe Form 1 in der Zusammensetzung vorliegt.

**17.** Zusammensetzung nach einem der Ansprüche 2 bis 11, wobei mehr als 95 % der Verbindung I (nach Gewicht) als amorphe Form 1 in der Zusammensetzung vorliegt.

**18.** Zusammensetzung nach einem der Ansprüche 2 bis 11, wobei mehr als 97 % der Verbindung I (nach Gewicht) als amorphe Form 1 in der Zusammensetzung vorliegt.

**19.** Zusammensetzung nach einem der Ansprüche 2 bis 11, wobei mehr als 99 % der Verbindung I (nach Gewicht) als amorphe Form 1 in der Zusammensetzung vorliegt.

**20.** Kit, welches umfasst:

eine pharmazeutische Zusammensetzung, welche die Verbindung I mit der Formel

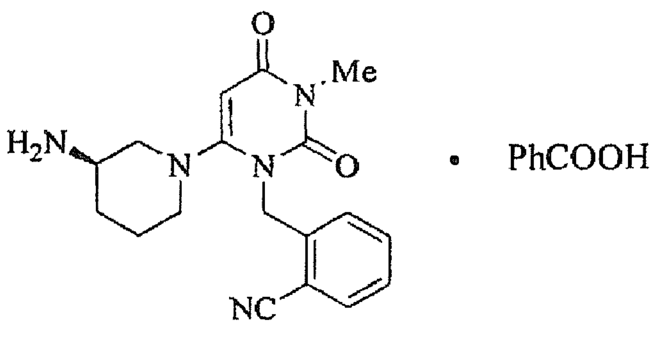

umfasst,

wobei wenigstens ein Teil der Verbindung I als amorphe Form 1 in der pharmazeutischen Zusammensetzung vorliegt, **gekennzeichnet durch** eine oder mehrere physikalische Eigenschaften, die ausgewählt sind aus der Gruppe bestehend aus:

einem Röntgenpulverbeugungsdiagramm unter Verwendung von Cu-Kα-Strahlung, das ein breites Halogen zeigt, wobei keine spezifischen Maxima vorliegen,
einem IR-Spektrum, welches Absorptionsmaxima bei 1703, 1599, 1119, 868 und 809 $cm^{-1}$ umfasst,
einem FT-Raman-Spektrum, welches Maxima bei 805, 1280 und 1703 $cm^{-1}$ umfasst, und

einem Differentialscanningkalorimetrie-Spektrum mit *Tg* = 70 °C (Beginn), einem Exothermen bei 132 °C und einem endothermen Beginn bei 183 °C, und
Anweisungen, welche eine oder mehrere Informationsformen umfassen, die ausgewählt sind aus der Gruppe bestehend aus der Angabe eines Krankheitszustandes, bei welchem die Zusammensetzung verabreicht werden soll, Aufbewahrungsinformationen für die Zusammensetzung, Dosierungsinformationen und Anweisungen, wie die Zusammensetzung verabreicht werden soll.

**21.** Kit gemäß Anspruch 20, wobei wenigstens ein Teil der Verbindung I als amorphe Form 1 in der pharmazeutischen Zusammensetzung vorliegt, **gekennzeichnet durch** eine oder mehrere physikalische Eigenschaften, die ausgewählt sind aus der Gruppe bestehend aus:

einem Röntgenpulverbeugungsdiagramm unter Verwendung von Cu-K$\alpha$-Strahlung, das ein breites Halogen zeigt, wobei keine spezifischen Maxima vorliegen,
einem IR-Spektrum, welches Absorptionsmaxima bei 3062, 2949, 2861, 2571, 2225, 2136, 1703, 1652, 1599, 1541, 1440, 1375, 1286, 1228, 1172, 1134, 1119, 1084, 1068, 1024, 948, 868, 833 und 809 $cm^{-1}$ umfasst,
einem FT-Raman-Spektrum, welches Maxima bei 805, 834, 904, 1002, 1024, 1045, 1134, 1168, 1205, 1280, 1386, 1443, 1578, 1600, 1654, 1703, 2225, 2864, 2958 und 3065 $cm^{-1}$ umfasst, und
einem Differentialscanningkalorimetrie-Spektrum mit *Tg* = 70 °C (Beginn), einem Exothermen bei 132 °C und einem endothermen Beginn bei 183 °C, und
Anweisungen, welche eine oder mehrere Informationsformen umfassen, die ausgewählt sind aus der Gruppe bestehend aus der Angabe eines Krankheitszustandes, bei welchem die Zusammensetzung verabreicht werden soll, Aufbewahrungsinformationen für die Zusammensetzung, Dosierungsinformationen und Anweisungen, wie die Zusammensetzung verabreicht werden soll.

**22.** Kit gemäß einem der Ansprüche 20 und 21, wobei das Kit die Zusammensetzung in einer Mehrfachdosierungsform umfasst.

**23.** Herstellungsartikel, welcher umfasst:

eine Zusammensetzung gemäß einem der Ansprüche 2 bis 19 und
Verpackungsmaterialien.

**24.** Herstellungsartikel gemäß Anspruch 23, wobei das Verpackungsmaterial einen Behälter zur Aufnahme der Zusammensetzung umfasst.

**25.** Herstellungsartikel gemäß Anspruch 24, wobei der Behälter eine Beschriftung umfasst, welche eine oder mehrere Elemente angibt aus der Gruppe bestehend aus einem Krankheitszustand, bei welchem die Zusammensetzung verabreicht werden soll, Aufbewahrungsinformationen, Dosierungsinformationen und/oder Anweisungen, wie die Zusammensetzung verabreicht werden soll.

**26.** Herstellungsartikel gemäß Anspruch 23, wobei der Herstellungsartikel die Zusammensetzung in einer Mehrfachdosierungsform umfasst.

**27.** Zusammensetzung gemäß einem der Ansprüche 2 bis 19 für die Verwendung als Medikament.

**28.** Verwendung einer Zusammensetzung gemäß einem der Ansprüche 2 bis 19 bei der Herstellung eines Medikaments zur Behandlung von Diabetes.

**29.** Verwendung einer Zusammensetzung gemäß einem der Ansprüche 2 bis 19 bei der Herstellung eines Medikaments zur Behandlung von Diabetes vom Typ II.

**30.** Verwendung einer Zusammensetzung gemäß einem der Ansprüche 2 bis 19 bei der Herstellung eines Medikaments zur Behandlung oder Vorbeugung von Krebsmetastasen.

**31.** Zusammensetzung gemäß einem der Ansprüche 2 bis 19 zur Verwendung bei der Behandlung von Diabetes.

**32.** Zusammensetzung gemäß einem der Ansprüche 2 bis 19 zur Verwendung bei der Behandlung von Diabetes vom Typ II.

**33.** Zusammensetzung gemäß einem der Ansprüche 2 bis 19 zur Verwendung bei der Behandlung oder Vorbeugung von Krebsmetastasen.

**34.** Verfahren zur Herstellung der Verbindung I mit der Formel

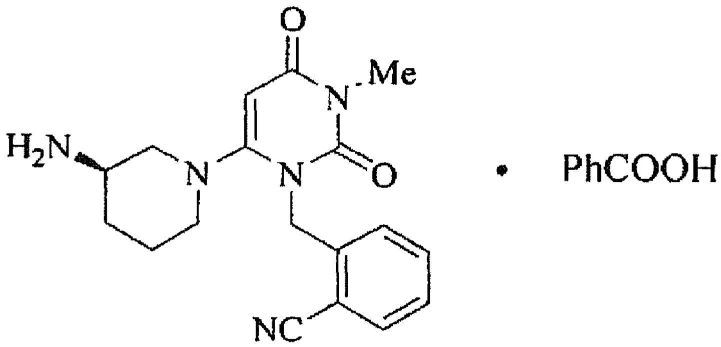

wobei wenigstens ein Teil der Verbindung I in amorpher Form 1 vorliegt und das Verfahren ausgewählt ist aus der Gruppe bestehend aus:

(a) Rotationsverdampfung aus Methanol,
(b) schneller Verdampfung aus Wasser,
(c) Lyophilisation aus Wasser,
(d) Kristallisation aus Ethylacetat und Hexanen, und Kristallisation aus Isopropylacetat und Hexanen, und Isolieren der Verbindung I aus dem Lösungsmittel.

## Revendications

**1.** Procédé de préparation d'un composé I de formule

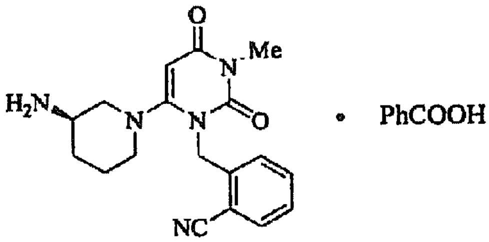

où au moins une partie du composé I est présente sous une forme polymorphe A **caractérisée par** une ou plusieurs propriétés physiques choisies dans le groupe constitué par:

un diagramme de diffraction de rayons X sur poudre, utilisant le rayonnement Kα du Cu, dont des caractéristiques saillantes sont les raies de diffraction majeures suivantes :

| Position (°2θ) | 9,44 | 10,84 | 17,82 | 18,75 | 25,87 | 28,52 |
|---|---|---|---|---|---|---|

un spectre IR comprenant des pics d'absorption à 1212, 1365, 1447, 1613 et 1697 $cm^{-1}$;
un spectre FT-Raman comprenant des pics à 1065, 1103, 1235, 1288, 1337, 1365, 1624, 1689, 2883, 2983 et 3026 $cm^{-1}$; et
un spectre de calorimétrie différentielle à balayage ayant une zone endothermique d'environ 173°C à environ 195°C,

le procédé comprenant:

(a) la cristallisation dans l'un quelconque des systèmes de solvants suivants, comprenant (i) de l'acétone, (ii) de l'acétonitrile; (iii) du butanol; (iv) du diméthylsulfoxyde; (v) du dioxane: (vi) de l'éthanol; (vii) de l'éthanol et de l'isopropanol; (viii) de l'éthanol et de l'eau; (ix) de l'acétate d'éthyle; (x) de l'heptane; (xi) de l'isopropanol; (xii) de l'acétate d'isopropyle; (xiii) du méthanol; (xiv) de la méthyléthylcétone; (xv) de la méthylisobutylcétone; (xvi) du 2,2,2-trifluoroéthanol; (xvii) du tétrahydrofurane; (xviii) du toluène; (xix) de l'eau; et (xx) de l'éthanol et de l'heptane; ou
(b) la conversion d'une forme amorphe 1 par soumission de la forme amorphe 1 à une contrainte par la chaleur, une humidité relativement élevée ou des vapeurs organiques, ou par broyage par voie humide de la forme amorphe 1 avec de l'eau.

**2.** Composition comprenant le composé I ayant la formule

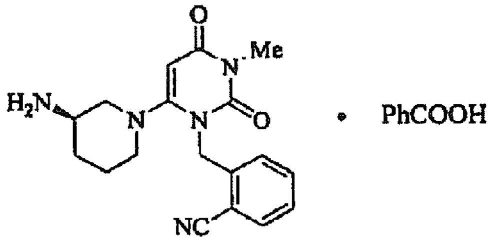

où au moins une partie du composé I est présente dans la composition sous la forme amorphe 1 **caractérisée par** une ou plusieurs propriétés physiques choisies dans le groupe constitué par:

un diagramme de diffraction de rayons X sur poudre, utilisant le rayonnement Kα du Cu, qui présente un large halo sans présence de pics spécifiques;
un spectre IR comprenant des pics d'absorption à 1703, 1599, 1119, 868 et 809 $cm^{-1}$;
un spectre FT-Raman comprenant des pics à 805, 1280 et 1703 $cm^{-1}$; et
un spectre de calorimétrie différentielle à balayage ayant une Tg = 70°C (début), un pic exothermique à 132°C et un pic endothermique débutant à 183°C.

**3.** Composition selon la revendication 2, où au moins une partie du composé I est présente dans la composition sous la forme amorphe 1 **caractérisée par** une ou plusieurs propriétés physiques choisies dans le groupe constitué par:

un diagramme de diffraction de rayons X sur poudre, utilisant le rayonnement Kα du Cu, qui présente un large halo sans présence de pics spécifiques ;
un spectre IR comprenant des pics d'absorption à 3062, 2949, 2861, 2571, 2225, 2136, 1703, 1652, 1599, 1541, 1440, 1375, 1286, 1228, 1172, 1134, 1119, 1084, 1068, 1024, 948, 868, 833 et 809 $cm^{-1}$;
un spectre FT-Raman comprenant des pics à 805, 834, 904, 1002, 1024, 1045, 1134, 1168, 1205, 1280, 1386, 1443, 1578, 1600, 1654, 1703, 2225, 2864, 2958 et 3065 $cm^{-1}$; et
un spectre de calorimétrie différentielle à balayage ayant une Tg = 70°C (début), un pic exothermique à 132°C et un pic endothermique débutant à 183°C.

**4.** Composition pharmaceutique comprenant:

un composé I de formule

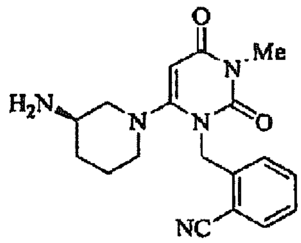 • PhCOOH

où au moins une partie du composé I est présente dans la composition pharmaceutique sous la forme amorphe 1 **caractérisée par** une ou plusieurs propriétés physiques choisies dans le groupe constitué par:

un diagramme de diffraction de rayons X sur poudre, utilisant le rayonnement Kα du Cu, qui présente un large halo sans présence de pics spécifiques;
un spectre IR comprenant des pics d'absorption à 1703, 1599, 1119, 868 et 809 $cm^{-1}$;
un spectre FT-Raman comprenant des pics à 805, 1280 et 1703 $cm^{-1}$; et
un spectre de calorimétrie différentielle à balayage ayant une Tg = 70°C (début), un pic exothermique à 132°C et un pic endothermique débutant à 183°C; et un ou plusieurs supports pharmaceutiques.

**5.** Composition pharmaceutique selon la revendication 4, où au moins une partie du composé I est présente dans la composition pharmaceutique sous la forme amorphe 1 **caractérisée par** une ou plusieurs propriétés physiques choisies dans le groupe constitué par:

un diagramme de diffraction de rayons X sur poudre, utilisant le rayonnement Kα du Cu, qui présente un large halo sans présence de pics spécifiques ;
un spectre IR comprenant des pics d'absorption à 3062, 2949, 2861, 2571, 2225, 2136, 1703, 1652, 1599, 1541, 1440, 1375, 1286, 1228, 1172, 1134, 1119, 1084, 1068, 1024, 948, 868, 833 et 809 $cm^{-1}$;
un spectre FT-Raman comprenant des pics à 805, 834, 904, 1002, 1024, 1045, 1134, 1168, 1205, 1280, 1386, 1443, 1578, 1600, 1654, 1703, 2225, 2864, 2958 et 3065 $cm^{-1}$; et
un spectre de calorimétrie différentielle à balayage ayant une Tg = 70°C (début), un pic exothermique à 132°C et un pic endothermique débutant à 183°C; et un ou plusieurs supports pharmaceutiques.

**6.** Composition pharmaceutique selon l'une quelconque des revendications 4 et 5, la composition étant une pilule ou une capsule adaptée pour l'administration orale.

**7.** Composition pharmaceutique selon l'une quelconque des revendications 4 et 5, la composition étant sous une forme galénique orale choisie dans le groupe constitué par des pilules, des comprimés, des capsules, des émulsions, des suspensions, des microsuspensions, des cachets, des pulvérisations, des gommes à mâcher, des poudres, des poudres lyophilisées, des granules et des pastilles.

**8.** Composition pharmaceutique selon l'une quelconque des revendications 4 et 5, la composition étant sous une forme galénique pour administration parentérale choisie dans le groupe constitué par des suspensions, des microsuspensions, des émulsions, des formes solides appropriées pour une mise en suspension ou un émulsionnement avant l'injection, et des dispositifs implantables.

**9.** Composition pharmaceutique selon l'une quelconque des revendications 4 et 5, la composition étant adaptée pour l'administration topique ou transdermique.

**10.** Composition pharmaceutique selon l'une quelconque des revendications 4 et 5, la composition étant sous une forme galénique topique ou transdermique choisie dans le groupe constitué par des suspensions, des microsuspensions, des émulsions, des crèmes, des gels, des pommades, des lotions, des teintures, des pâtes, des poudres, des mousses, des aérosols, des lavages, des pulvérisations, des suppositoires, des bandages et des timbres dermiques.

**11.** Composition pharmaceutique selon l'une quelconque des revendications 4 et 5, la composition étant sous une forme galénique pulmonaire choisie dans le groupe constitué par des poudres, des aérosols, des suspensions, des microsuspensions et des émulsions.

**12.** Composition selon l'une quelconque des revendications 2-11, où plus de 1 % du composé 1 (en masse) est présent dans la composition sous la forme amorphe 1.

**13.** Composition selon l'une quelconque des revendications 2-11, où plus de 5 % du composé 1 (en masse) sont présents dans la composition sous la forme amorphe 1.

**14.** Composition selon l'une quelconque des revendications 2-11, où plus de 50 % du composé 1 (en masse) sont présents dans la composition sous la forme amorphe 1.

**15.** Composition selon l'une quelconque des revendications 2-11, où plus de 75 % du composé 1 (en masse) sont présents dans la composition sous la forme amorphe 1.

**16.** Composition selon l'une quelconque des revendications 2-11, où plus de 90 % du composé 1 (en masse) sont présents dans la composition sous la forme amorphe 1.

**17.** Composition selon l'une quelconque des revendications 2-11, où plus de 95 % du composé 1 (en masse) sont présents dans la composition sous la forme amorphe 1.

**18.** Composition selon l'une quelconque des revendications 2-11, où plus de 97 % du composé 1 (en masse) sont présents dans la composition sous la forme amorphe 1.

**19.** Composition selon l'une quelconque des revendications 2-11, où plus de 99 % du composé 1 (en masse) sont présents dans la composition sous la forme amorphe 1.

**20.** Kit comprenant:

une composition pharmaceutique comprenant un composé I de formule

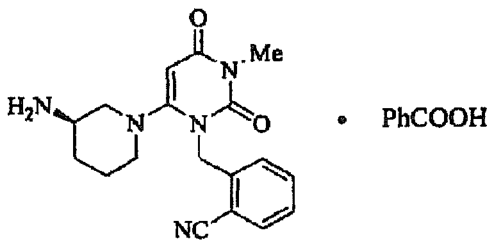

où au moins une partie du composé I est présente dans la composition pharmaceutique sous la forme amorphe 1 **caractérisée par** une ou plusieurs propriétés physiques choisies dans le groupe constitué par:

un diagramme de diffraction de rayons X sur poudre, utilisant le rayonnement Kα du Cu, qui présente un large halo sans présence de pics spécifiques ;
un spectre IR comprenant des pics d'absorption à 1703, 1599, 1119, 868 et 809 $cm^{-1}$;
un spectre FT-Raman comprenant des pics à 805, 1280 et 1703 $cm^{-1}$; et
un spectre de calorimétrie différentielle à balayage ayant une Tg = 70°C (début), un pic exothermique à 132°C et un pic endothermique débutant à 183°C; et
des instructions qui comprennent une ou plusieurs formes d'informations choisies dans le groupe constitué par l'indication d'un état pathologique pour lequel la composition sera administrée, des informations de conservation de la composition, des informations sur la posologie et des instructions concernant la manière d'administrer la composition.

**21.** Kit selon la revendication 20, dans lequel au moins une partie du composé I est présente dans la composition pharmaceutique sous la forme amorphe 1 **caractérisée par** une ou plusieurs propriétés physiques choisies dans le groupe constitué par:

un diagramme de diffraction de rayons X sur poudre, utilisant le rayonnement Kα du Cu, qui présente un large

halo sans présence de pics spécifiques ;
un spectre IR comprenant des pics d'absorption à 3062, 2949, 2861, 2571, 2225, 2136, 1703, 1652, 1599, 1541, 1440, 1375, 1286, 1228, 1172, 1134, 1119, 1084, 1068, 1024, 948, 868, 833 et 809 $cm^{-1}$;
un spectre FT-Raman comprenant des pics à 805, 834, 904, 1002, 1024, 1045, 1134, 1168, 1205, 1280, 1386, 1443, 1578, 1600, 1654, 1703, 2225, 2864, 2958 et 3065 $cm^{-1}$; et
des instructions qui comprennent une ou plusieurs formes d'informations choisies dans le groupe constitué par l'indication d'un état pathologique pour lequel la composition sera administrée, des informations de conservation de la composition, des informations sur la posologie et des instructions concernant la manière d'administrer la composition.

**22.** Kit selon l'une quelconque des revendications 20 et 21, le kit comprenant la composition sous une forme de doses multiples.

**23.** Article manufacturé comprenant:

une composition selon l'une quelconque des revendications 2-19; et
du matériel d'emballage.

**24.** Article manufacturé selon la revendication 23, dans lequel le matériel d'emballage comprend un récipient pour loger la composition.

**25.** Article manufacturé selon la revendication 24, dans lequel le récipient comprend une étiquette indiquant un ou plusieurs éléments du groupe constitué par un état pathologique pour lequel la composition sera administrée, des informations sur la conservation, des informations sur la posologie et/ou des instructions concernant la manière d'administrer la composition.

**26.** Article manufacturé selon la revendication 23, l'article manufacturé comprenant la composition sous forme de doses multiples.

**27.** Composition selon l'une quelconque des revendications 2-19, à utiliser comme médicament.

**28.** Utilisation d'une composition selon l'une quelconque des revendications 2-19 dans la préparation d'un médicament destiné au traitement du diabète.

**29.** Utilisation d'une composition selon l'une quelconque des revendications 2-19 dans la préparation d'un médicament destiné au traitement du diabète de type II.

**30.** Utilisation d'une composition selon l'une quelconque des revendications 2-19 dans la préparation d'un médicament destiné au traitement ou à la prévention de métastases cancéreuses.

**31.** Composition selon l'une quelconque des revendications 2-19, à utiliser dans le traitement du diabète.

**32.** Composition selon l'une quelconque des revendications 2-19, à utiliser dans le traitement du diabète de type II.

**33.** Composition selon l'une quelconque des revendications 2-19, à utiliser dans le traitement ou la prévention de métastases cancéreuses.

**34.** Procédé de préparation du composé 1 ayant la formule

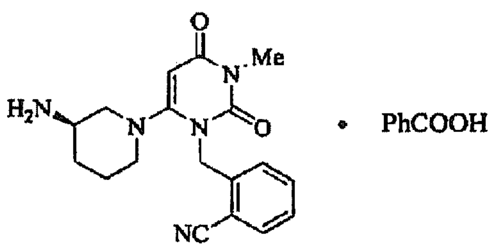

au moins une partie du composé I étant présente sous la forme amorphe 1, le procédé étant choisi dans le groupe constitué par:

(a) l'évaporation à l'évaporateur rotatif à partir du méthanol;
(b) l'évaporation rapide à partir d'eau;
(c) la lyophilisation à partir d'eau;
(d) la cristallisation dans de l'acétate d'éthyle et de l'hexane; et la cristallisation dans de l'acétate d'isopropyle et de l'hexane, et l'isolement du composé 1 à partir du solvant.

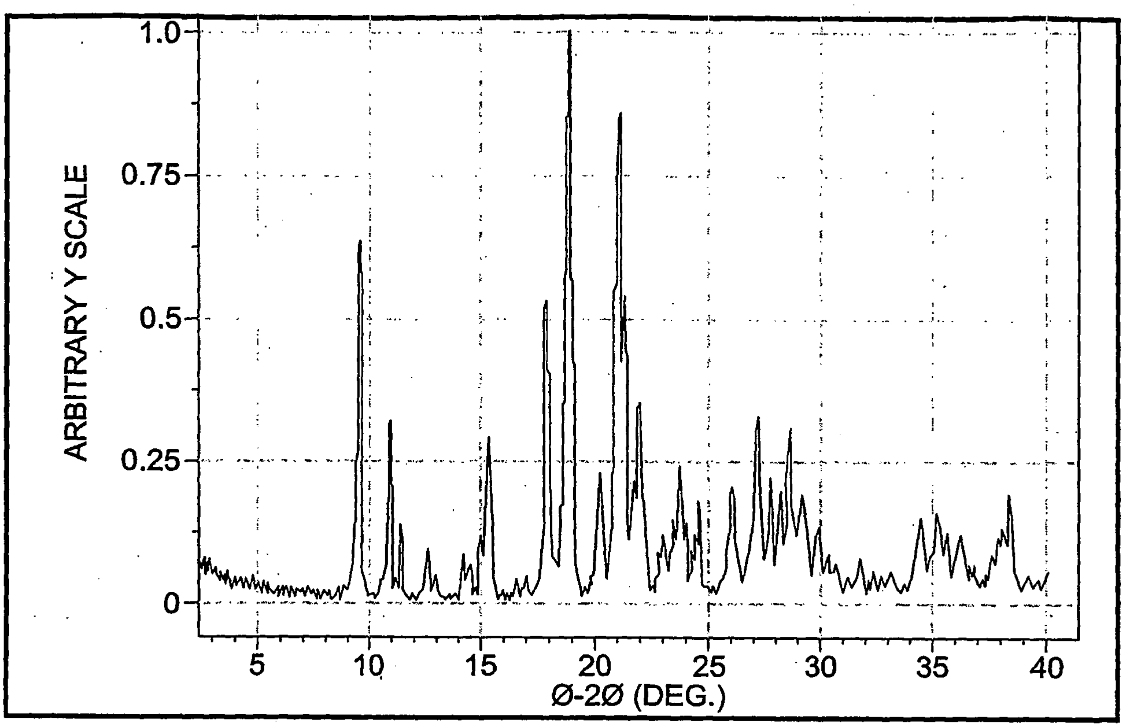
ARBITRARY Y SCALE
1.0
0.75
0.5
0.25
0
5
10
15
20
25
30
35
40
Ø-2Ø (DEG.)

FIG. 1

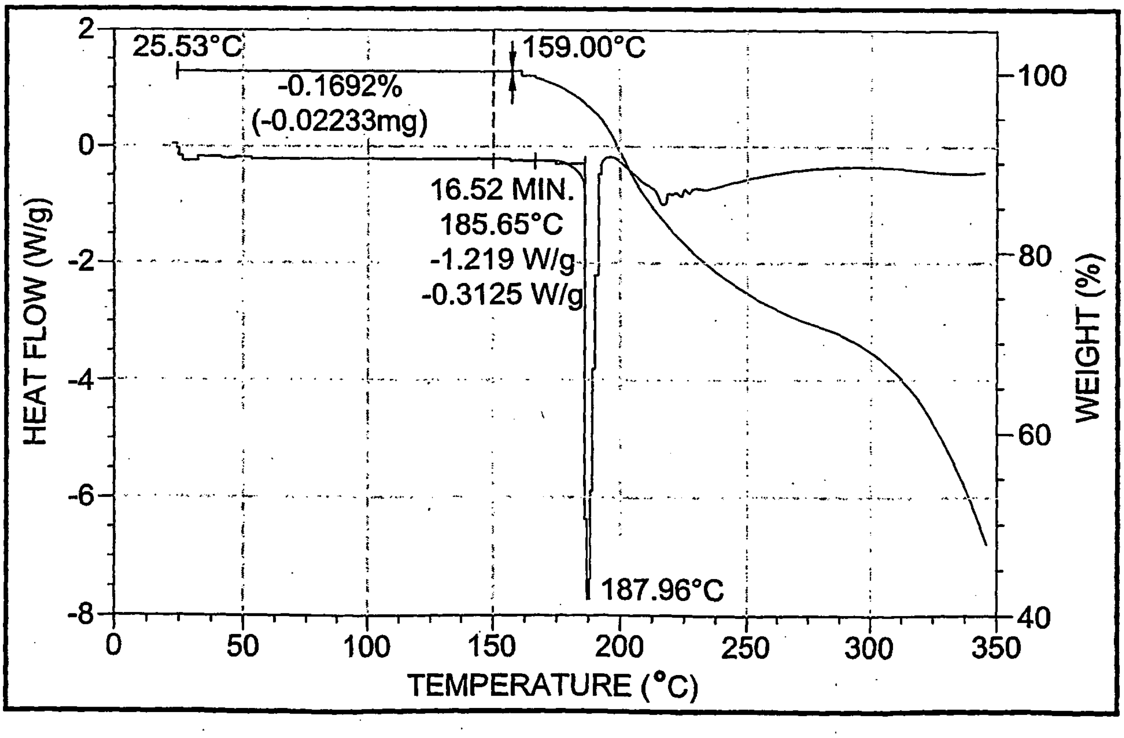
HEAT FLOW (W/g)
WEIGHT (%)
25.53°C
159.00°C
-0.1692%
(-0.02233mg)
16.52 MIN.
185.65°C
-1.219 W/g
-0.3125 W/g
187.96°C
2
0
-2
-4
-6
-8
100
80
60
40
0
50
100
150
200
250
300
350
TEMPERATURE (°C)

FIG. 2

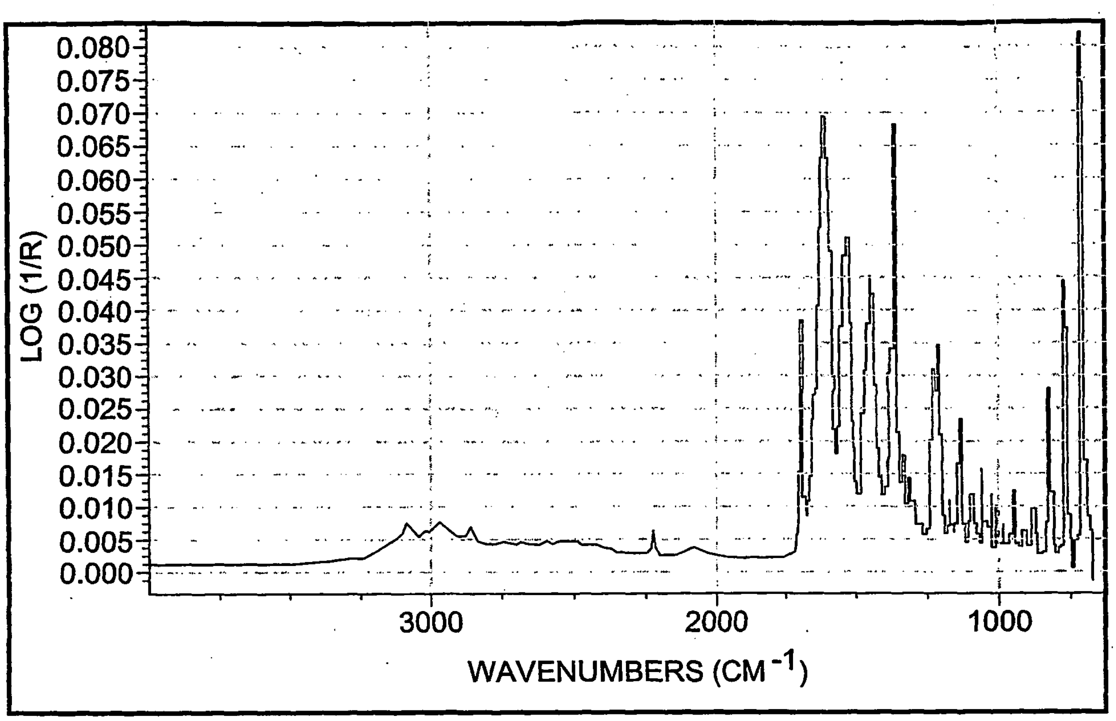
0.080
0.075
0.070
0.065
0.060
0.055
0.050
0.045
0.040
0.035
0.030
0.025
0.020
0.015
0.010
0.005
0.000
LOG (1/R)
3000
2000
1000
WAVENUMBERS (CM$^{-1}$)

FIG. 3

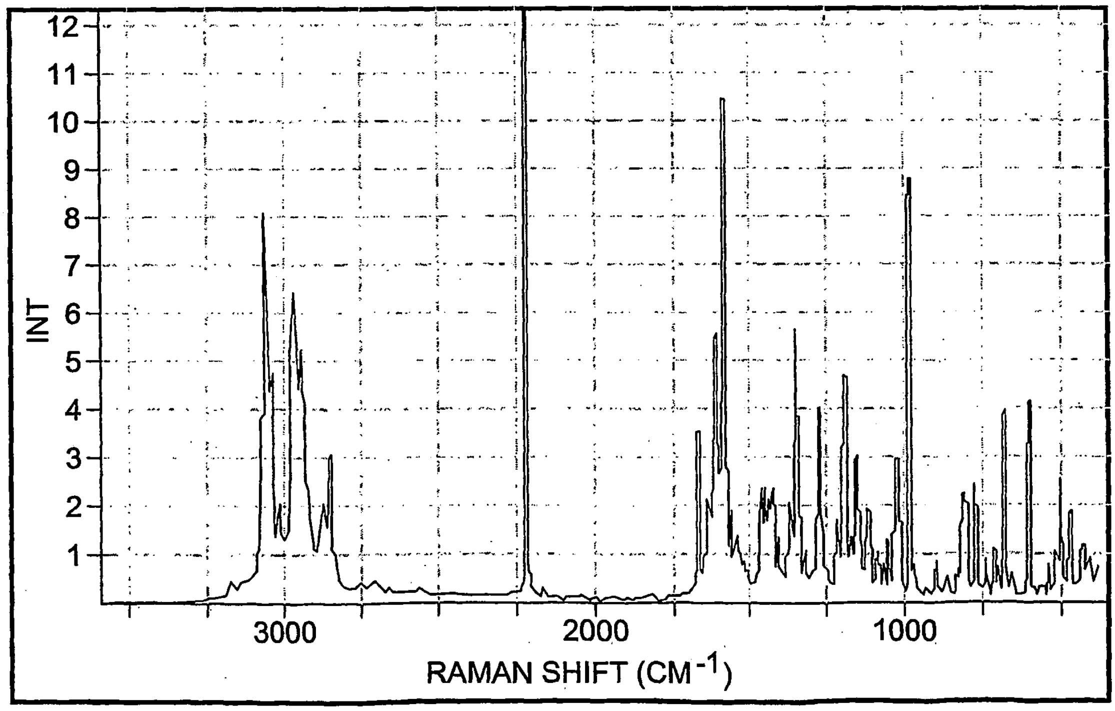
12
11
10
9
8
7
6
5
4
3
2
1
INT
3000
2000
1000
RAMAN SHIFT (CM$^{-1}$)

FIG. 4

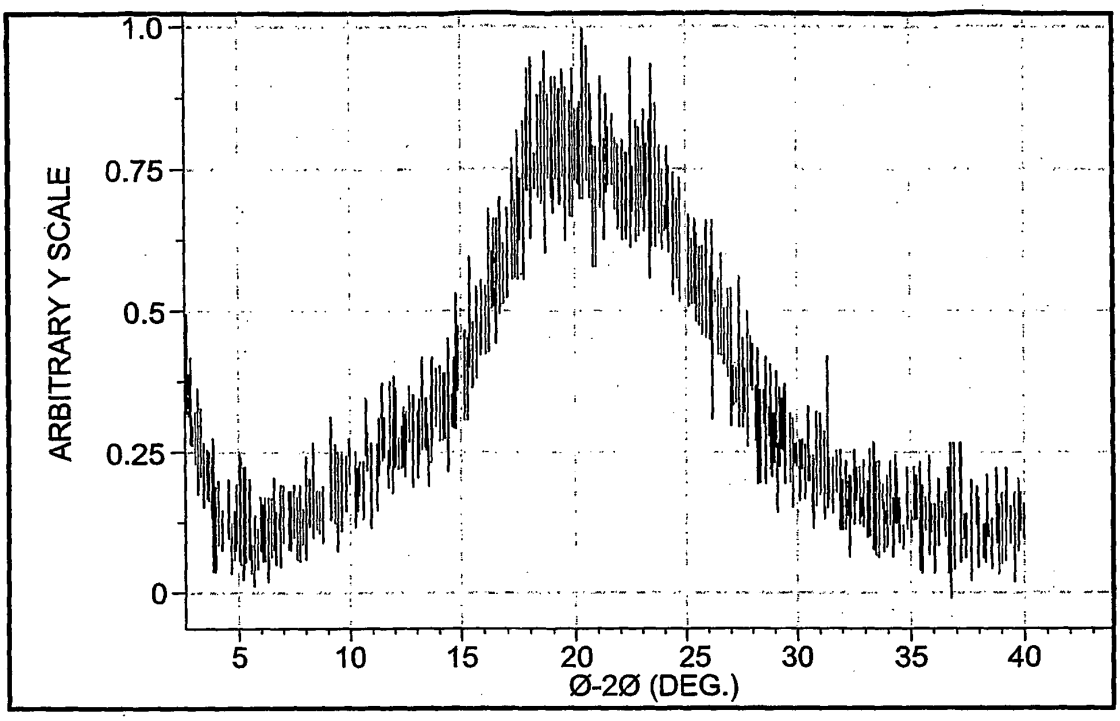
ARBITRARY Y SCALE
1.0
0.75
0.5
0.25
0
5
10
15
20
25
30
35
40
Ø-2Ø (DEG.)

FIG. 5

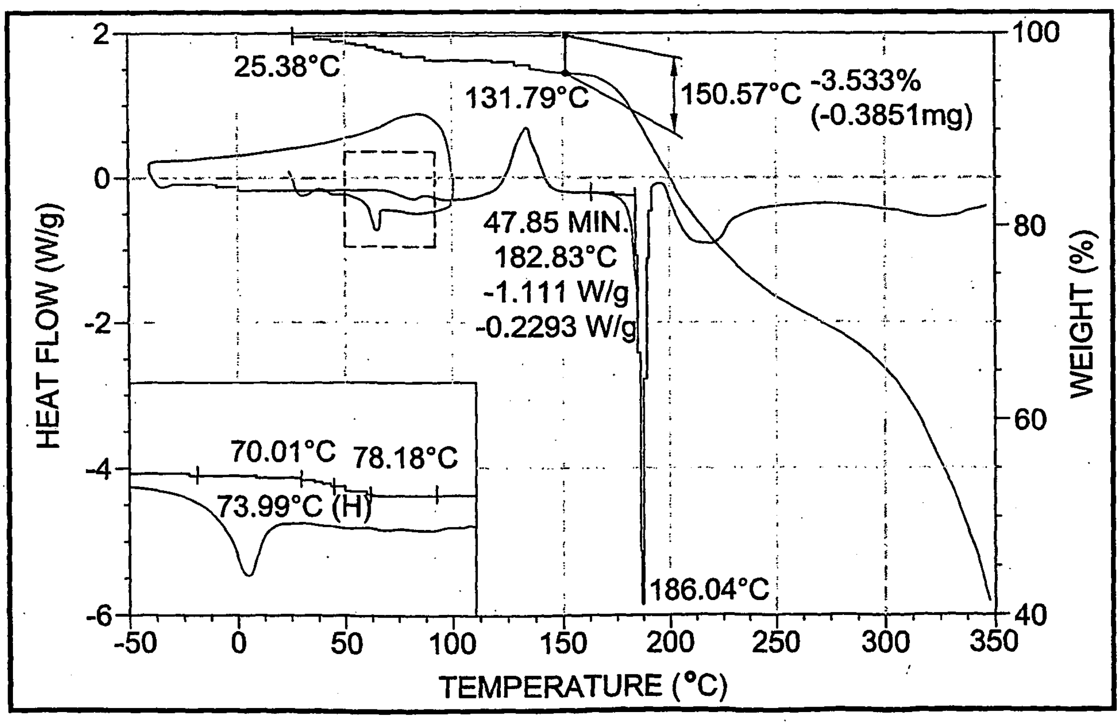
HEAT FLOW (W/g)
WEIGHT (%)
25.38°C
131.79°C
150.57°C
-3.533%
(-0.3851mg)
47.85 MIN.
182.83°C
-1.111 W/g
-0.2293 W/g
70.01°C
78.18°C
73.99°C (H)
186.04°C
TEMPERATURE (°C)

FIG. 6

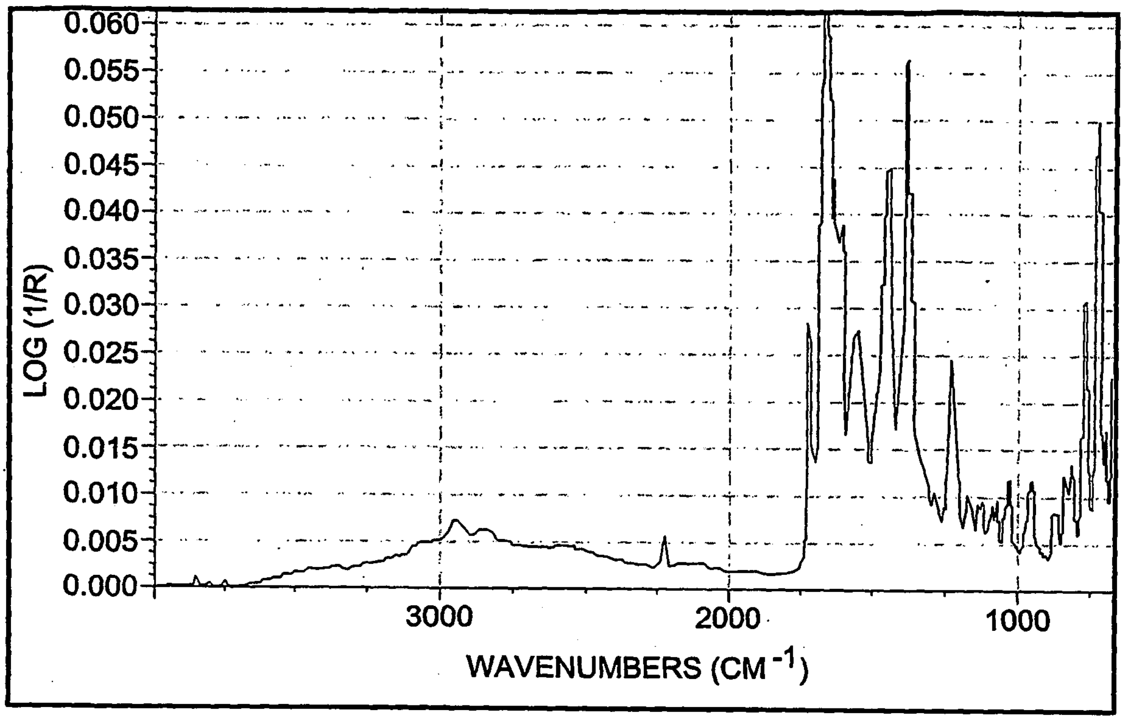
0.060
0.055
0.050
0.045
0.040
0.035
0.030
0.025
0.020
0.015
0.010
0.005
0.000
LOG (1/R)
3000
2000
1000
WAVENUMBERS (CM$^{-1}$)

FIG. 7

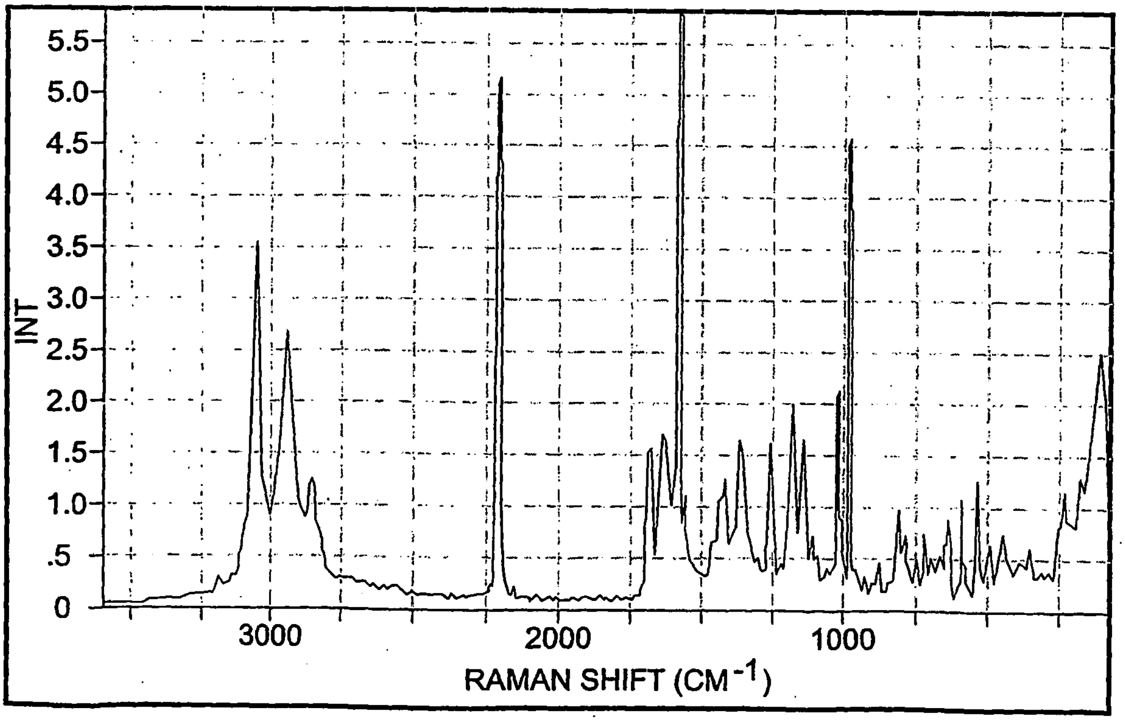
5.5
5.0
4.5
4.0
3.5
3.0
2.5
2.0
1.5
1.0
.5
0
INT
3000
2000
1000
RAMAN SHIFT (CM$^{-1}$)

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 93017173 W
- WO 9403055 A
- US 08099205 A


### Non-patent literature cited in the description


- **Mattem, T. et al.** *Scand. J. Immunol.,* 1991, vol. 33, 737
- **Schon, E. et al.** *Biol. Chem.,* 1991, vol. 372, 305
- **Schon, E. et al.** *Scand. J. Immunol.,* 1989, vol. 29, 127
- **Flentke, G. R. et al.** *Proc. Nat. Acad. Sci. USA,* 1991, vol. 88, 1556
- **Kubota, T. et al.** *Clin. Exp. Immun.,* 1992, vol. 89, 192
- **Wakselman, M. ; Nguyen, C. ; Mazaleyrat, J.-P. ; Callebaut, C. ; Krust, B. ; Hovanessian, A. G.** Inhibition of HIV-1 infection of CD 26+ but not CD 26-cells by a potent cyclopeptidic inhibitor of the DPP-IV activity of CD 26. *Abstract P.44 of the 24th European Peptide Symposium,* 1996
- **Kameoka, J. et al.** *Science,* 1900, vol. 26, 466
- **Johnson, R. C. et al.** *J. Cell. Biol.,* 1993, vol. 121, 1423
- **Raynaud, F. et al.** *J. Cell. Physiol.,* 1992, vol. 151, 378
- **Vanhoof, G. et al.** *Eur. J. Clin. Chem. Clin. Biochem.,* 1992, vol. 30, 333
- **Mentlein, R. ; Dahms, P. ; Grandt, D. ; Kruger, R.** Proteolytic processing of neuropeptide Y and peptide YY by dipeptidyl peptidase IV. *Regul. Pept.,* 1993, vol. 49, 133
- **Wetzel, W. ; Wagner, T. ; Vogel, D. ; Demuth, H.-U. ; Balschun, D.** Effects of the CLIP fragment ACTH 20-24 on the duration of REM sleep episodes. *Neuropeptides,* 1997, vol. 31, 41
- **J. March.** Advanced Organic Chemistry: Reactions, Mechanisms and Structure. Wiley-Interscience, 1992
- Remington's Pharmaceutical Sciences. Mack Publishing Company, 1995
- U.S. Pharmacopoeia. U.S.P. Pharmacopeial Convention, Inc, vol. 24